(19) Europäisches Patentamt
European Patent Office
Office européen des brevets

(11) EP 3 018 214 B1

(12) **EUROPEAN PATENT SPECIFICATION**

(45) Date of publication and mention of the grant of the patent:
**13.12.2017 Bulletin 2017/50**

(51) Int Cl.:
***C12Q 1/68*** *(2006.01)*

(21) Application number: **14191967.0**

(22) Date of filing: **05.11.2014**

(54) **Method for quantifying the level of minimal residual disease in a subject**

Verfahren zur Quantifizierung des Niveaus minimaler Resterkrankung bei einer Person

Procédé pour quantifier le niveau de maladie résiduelle minimale chez un sujet

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.05.2016 Bulletin 2016/19**

(73) Proprietor: **Fundacion de Investigacion Hospital 12 de Octubre
28041 Madrid (ES)**

(72) Inventors:
- **Barrio García, Santiago
Madrid (ES)**
- **Martínez López, Joaquín
Madrid (ES)**
- **Marín Sebastián, Carlos
Madrid (ES)**
- **Rapado Martínez, María Inmuaculada
Madrid (ES)**
- **Ayala Díaz, Rosa María
Madrid (ES)**
- **Sánchez Vega Carrión, Beatriz
28041 Madrid (ES)**

(74) Representative: **Manuel Illescas Asociados, S.L.
Príncipe de Vergara 197, Oficina 1°A
28002 Madrid (ES)**

(56) References cited:
**WO-A1-2014/062945 US-A1- 2014 235 454**

- **M. FAHAM ET AL: "Deep-sequencing approach for minimal residual disease detection in acute lymphoblastic leukemia", BLOOD, vol. 120, no. 26, 20 December 2012 (2012-12-20), pages 5173-5180, XP055172854, ISSN: 0006-4971, DOI: 10.1182/blood-2012-07-444042**
- **J. MARTINEZ-LOPEZ ET AL: "Prognostic value of deep sequencing method for minimal residual disease detection in multiple myeloma", BLOOD, vol. 123, no. 20, 15 May 2014 (2014-05-15) , pages 3073-3079, XP055173548, ISSN: 0006-4971, DOI: 10.1182/blood-2014-01-550020**
- **MATHIEU GIRAUD ET AL: "Fast multiclonal clusterization of V(D)J recombinations from high-throughput sequencing", BMC GENOMICS, BIOMED CENTRAL LTD, LONDON, UK, vol. 15, no. 1, 28 May 2014 (2014-05-28), page 409, XP021187326, ISSN: 1471-2164, DOI: 10.1186/1471-2164-15-409**
- **D. Wu ET AL: "Detection of Minimal Residual Disease in B Lymphoblastic Leukemia by High-Throughput Sequencing of IGH", CLINICAL CANCER RESEARCH, vol. 20, no. 17, 26 June 2014 (2014-06-26) , pages 4540-4548, XP055336751, US ISSN: 1078-0432, DOI: 10.1158/1078-0432.CCR-13-3231**

EP 3 018 214 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention may be included in the field of medicine in general, more particularly in the field of diagnosis of disease.

In particular, the present invention is focused on a method and kit for quantifying the level of minimal residual disease in a subject. In addition, the present invention is focussed on use of the method and/or kit for quantifying the level of minimal residual disease in a subject.

### Background to the invention

Current methods for the detection and treatment of disease mean that it is possible to control many diseases at a clinical level, thereby obliterating all traces of the disease. Nevertheless, it may be that some diseases are not detected or that the subject's recovery is not complete after treatment. In the latter case, the disease may develop drug resistance under selective pressure of treatment by a process of clonal selection, thus allowing expansion and ultimately disease recidivism or relapse. It is therefore very important to monitor the number of diseased cells in a given tissue. In particular, it is especially beneficial to monitor the level of minimal residual disease (MRD) in subjects who have been treated for a disease. MRD is the name given to the disease that remains in a subject or a particular tissue thereof during or after treatment of said disease. Typically, MRD refers to the cancer and/or leukaemia that remains in a subject during or after treatment of said cancer and/or leukaemia by, for example, chemotherapy.

Currently monitoring through, for example, determination of MRD may be performed by different techniques. Firstly, flow cytometry (FCM) can use up to 8 different markers to determine the disease phenotype. Secondly, another method used to this end is allele specific oligonucleotide PCR (ASO-PCR) of immunoglobulin (Igs) genes, which requires the design of specific primers for each patient or a specific molecular marker and is applicable only to 40% of cases. In this regard, WO 2004033728 A2 details primers for the amplification of immunoglobulin rearrangements for the diagnosis of lymphoproliferative disorders. The amplification by PCR of rearranged immunoglobulins or genes has also been used in US 7785783 B2 to characterize nucleic acid regions based on the identification of regions flanking a marker nucleic acid region. This provides means of analyzing a marker which is characteristic of a clonal population of cells and for monitoring the progression of a condition.
Finally, the emergence of next-generation sequencing (NGS) technologies has made it possible to analyze millions of sequences at once, each coming from different fragments of genome, or the result of amplification of the same region, representing a large number of cells. As a consequence, NGS technology may be used to identify disease and/or quantify a level of disease in subjects. In his regard, deep sequencing has been revealed as an alternative methodology to the aforementioned techniques which provides greater applicability than ASO-PCR and higher sensitivity than FCM [cf. Martinez-Lopez J. et al. "Prognostic value of deep sequencing method for minimal residual disease detection in multiple myeloma"; Blood (2014) 123(20):3073-3079]. This technique has also proved useful for detecting and quantifying myeloma cells in both the bone marrow and the peripheral blood [*cf.* Vij R. et al. "Deep sequencing reveals myeloma cells in peripheral blood in majority of multiple myeloma patients"; J. Clin. Lymphoma Myeloma Leuk. (2013) 14(2):131-139].

In addition, NGS technology has made it possible to identify clonogenic B or T cells with high sensitivity and specificity, thus allowing the detection of minimal residual disease in conditions as acute lymphoblastic leukemias (ALL), mantle cell lymphoma (MCL) and multiple myeloma (MM) [*cf.* Ladetto M. et al. "Next-generation sequencing and real-time quantitative PCR for minimal residual disease detection in B-cell disorders"; Leukemia (2014) 28(6):1299-1307]. NGS has also proven to be a useful method for the identification of clonotypic profiles to detect and monitor a disease from a lymphocyte sample. US 8628927 B2 demonstrates that the MRD of ALL can be established by comparing the sequence of a sample to previously-obtained clonotype profiles and markers on leukemic cells. In addition, another approach for the detection of MRD is the determination of a clonotype profile by the use of sequence tags for producing sequence-based profiles of complex nucleic-acid populations, as disclosed in WO 2013188471 A2. Faham et al. (Blood (2012) 120(26) 5173-5180) described a deep sequencing approach for minimal residual disease detection in ALL which relies on the addition of a known quantity of reference IgH. Although the aforementioned documents disclose several different approaches for detecting MRD wherein improvements in are achieved through different methods of sequencing, none of these documents specifically discloses a method which achieves improvement through the alignment strategy used. The method of alignment used is important to the accuracy of any method which is based on comparison of nucleotide sequences because the rate of failures of sequencers using a classical binary logic - in which sequences can only be equal or different - is so high that it is not useful. In fact, a high proportion (nearly all) of nucleotide sequences that evaluate as different, are equal but appear as different because of an error in the sequencer. In this regard, US 8628927 B2 mentions that some sequences are harder to align than others due to somatic mutations and diverse regions [such as the NDN region between the variable (V) and joining (J) gene segments in lymphocytes]. However, despite generally disclosing that alignment may be achieved using references sequences such as primer binding sequences or non-reference sequences, this document does not disclose a specific method for alignment that is capable of determining the level of disease in a subject irrespective of the genetic characteristics of the nucleotide or the disease.

It is the problem of the present invention to provide a method for the quantification of the level of disease in a patient, wherein said method exhibits improved sensitivity, greater reproducibility and more accurate determination of said levels. It is a further problem of the present invention to provide a universal method which is capable of determining the level of disease in a subject irrespective of the genetic characteristics of the nucleotide or the disease. In addition, it is a problem of the present invention to provide a method which is subject-specific and does not require access to external databases comprising data obtained from populations of subjects. Moreover, it is a problem of the invention to provide a method for the quantification of tumor clonotypic sequences for immunoglobulin gene rearrangements, as well as allelic load, point mutations (SNV), multiple mutations (MNV), indels, long insertions and translocations.

**Brief description of the invention**

The present invention discloses a method for quantifying the level of minimal residual disease (MRD) in a subject who has been treated for said disease, which comprises:

(a)

- amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- sequencing said at least one nucleotide sequence to obtain at least one first list of characters reading from left to right;

(b)

- amplifying by polymerase chain reaction using the same primers as in step (a), at least one nucleotide sequence in a biological sample obtained from said subject prior to treatment for said disease; and
- sequencing said at least one nucleotide sequence to obtain at least one second list of characters reading from left to right;

(c) determining, for each first list of characters obtained in step (a), the degree of similarity with each second list of characters obtained in step (b), wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and

- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and
(xviii) calculating DS according to the following formula:

$$DS = C_c / C_t$$

(d) selecting, for each first list of characters obtained in step (a), the DS of highest value, $DS_{HV}$;
(e) adding up the number of first lists of characters which have a $DS_{HV}$ that is greater than a threshold value, T, to obtain the total number of first lists of characters, $L_c$, which are the same as a second list of characters;
(f) adding up

- $L_c$; and
- the number of first lists of characters which do not have a $DS_{HV}$ that is greater than T,

to obtain the total number of first lists of characters, $L_t$; and
(g) calculating the level of minimal residual disease (MRD) according to the following formula:

$$MRD = L_c \times (D / k) / L_t^2.$$

The invention further relates to a kit and its use which is defined in the appended claims 8-15. In addition the present disclosure contains a kit for quantifying the level of minimal residual disease (MRD) in a subject who has been treated for said disease which comprises:

(a)

- means for amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- means for sequencing said at least one nucleotide sequence to obtain at least one first list of characters reading from left to right;

(b)

- means for amplifying by polymerase chain reaction using the same primers as in step (a), at least one nucleotide sequence in a biological sample obtained from said subject prior to treatment for said disease; and
- means for sequencing said at least one nucleotide sequence to obtain at least one second list of characters reading from left to right;

(c) means for determining, for each first list of characters obtained in step (a), the degree of similarity with each second list of characters obtained in step (b), wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and
(xviii) calculating DS according to the following formula:

$$DS = C_c / C_t$$

(d) means for selecting, for each first list of characters obtained in step (a), the DS of highest value, $DS_{HV}$;
(e) means for adding up the number of first lists of characters which have a $DS_{HV}$ that is greater than a threshold value, T, to obtain the total number of first lists of characters, $L_c$, which are the same as a second list of characters;
(f) means for adding up

- $L_c$; and
- the number of first lists of characters which do not have a $DS_{HV}$ that is greater than T,

to obtain the total number of first lists of characters, $L_t$; and
(g) means for calculating the level of minimal residual disease (MRD) according to the following formula:

$$MRD = L_c \times (D / k) / L_t^2.$$

Furthermore, the present invention discloses a use of the method disclosed herein or the kit disclosed herein in quantifying the level of minimal residual disease (MRD) in a subject who has been treated for said disease.

Moreover, the present invention relates to a method for quantifying the level of minimal residual disease (MRD) in a subject who has been treated for said disease, which comprises:

(a)

- amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- sequencing said at least one nucleotide sequence to obtain at least one first list of characters reading from left to right;

(b)

- amplifying by polymerase chain reaction using the same primers as in step (a), at least one nucleotide sequence in a biological sample obtained from said subject prior to treatment for said disease; and
- sequencing said at least one nucleotide sequence to obtain at least one second list of characters reading from left to right;

(c) determining, for each first list of characters obtained in step (a), the degree of similarity with each second list of characters obtained in step (b), wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and

(xviii) calculating DS according to the following formula:

$$DS = C_c / C_t$$

(d)

- amplifying by polymerase chain reaction using primers, each at least one nucleotide sequence which is the reverse complementary sequence complementary to the at least one nucleotide sequence in step (a), and sequencing said at least one reverse complementary nucleotide sequence to obtain at least one reverse complementary first list of characters reading from left to right; and
- amplifying by polymerase chain reaction using the same primers as in the previous step, each at least one nucleotide sequence which is the reverse complementary sequence complementary to the at least one nucleotide sequence in step (b), and sequencing said at least one reverse complementary nucleotide sequence to obtain at least one reverse complementary second list of characters reading from left to right; and
- determining, for each reverse complementary first list of characters obtained in step (a), the degree of similarity with each reverse complementary second list of characters obtained in step (b), wherein a degree of similarity, $DS_{rcs}$, of a reverse complementary first list of characters obtained in step (a) with a reverse complementary second list of characters obtained in step (b) is determined either by:

  (xix) selecting the character or longest continuous sequence of characters which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected;
  (xx) excluding the character or longest continuous sequence of characters selected in step (xix) from subsequent steps of selecting a character or longest continuous sequence of characters which is the same in the reverse complementary first list of characters and the reverse complementary second list of characters;
  (xxi)

  - selecting the character or longest continuous sequence of characters which is located to the left of the character or longest continuous sequence of characters excluded in step (xx) which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
  - selecting the character or longest continuous sequence of characters which is located to the right of the character or longest continuous sequence of characters excluded in step (xx) which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

  (xxii) excluding each character and/or each longest continuous sequence of characters selected in step (xxi) from subsequent steps of selecting a character or longest continuous sequence of characters which is the same in the reverse complementary first list of characters and the reverse complementary second list of characters;
  (xxiii)

  - selecting the character or longest continuous sequence of characters which is located in the continuous sequence of characters to the immediate left of each character or each longest continuous sequence of characters excluded in the previous step which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
  - selecting the character or longest continuous sequence of characters which is located in the continuous sequence of characters to the immediate right of each character or each longest continuous sequence of characters excluded in the previous step which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxiv) excluding each character and/or each longest continuous sequences of characters selected in step (xxiii) from subsequent steps of selecting a character or longest continuous sequence of characters which is the same in the reverse complementary first list of characters and the reverse complementary second list of characters;
(xxv) repeating steps (xxiii) and (xxiv) until no character or longest continuous sequence of characters which is the same in the reverse complementary first list of characters and the reverse complementary second list of characters is selected;
(xxvi) adding up

- the number of characters in the reverse complementary first list of characters which were excluded in any of the steps (xix) to (xxv); and
- the number of characters in the reverse complementary second list of characters which were excluded in any of the steps (xix) to (xxv) to obtain the total number of characters, $C_c$, in the reverse complementary first and second lists of characters which are the same as in the reverse complementary second and first lists of characters, respectively;

(xxvii) adding up

- $C_c$; and
- the number of characters in the reverse complementary first list of characters which are located between the characters and/or longest continuous sequences of characters that were excluded from the reverse complementary first list of characters, and which were not excluded in any of the steps (xix) to (xxv) of step (c); and
- the number of characters in the reverse complementary second list of characters which are located between the characters and/or longest continuous sequences of characters that were excluded from the reverse complementary second list of characters, and which were not excluded in any of the steps (xix) to (xxv) of step (c),

to obtain the total number of characters, $C_t$, in the reverse complementary first and second lists of characters; and
(xxviii) calculating DS according to the following formula:

$$DS_{rcs} = C_c / C_t$$

or by:

(xxix) selecting the character or longest continuous sequence of characters which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected;
(xxx) excluding the character or longest continuous sequence of characters selected in step (xxix) from subsequent steps of selecting a character or longest continuous sequence of characters which is the same in the reverse complementary first list of characters and the reverse complementary second list of characters;
(xxxi)

- selecting the character or longest continuous sequence of characters which is located to the left of the character or longest continuous sequence of characters excluded in step (xxx) which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and

- selecting the character or longest continuous sequence of characters which is located to the right of the character or longest continuous sequence of characters excluded in step (xxx) which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxxii) excluding each character and/or each longest continuous sequence of characters selected in step (xxxi) from subsequent steps of selecting a character or longest continuous sequence of characters which is the same in the reverse complementary first list of characters and the reverse complementary second list of characters;
(xxxiii) repeating steps (xxxi) and (xxxii) until no character or longest continuous sequence of characters which is the same in the reverse complementary first list of characters and the reverse complementary second list of characters is selected;
(xxxiv) adding up

- the number of characters in the reverse complementary first list of characters which were excluded in any of the steps (xxix) to (xxxiii); and
- the number of characters in the reverse complementary second list of characters which were excluded in any of the steps (xxix) to (xxxiii) to obtain the total number of characters, $C_c$, in the reverse complementary first and second lists of characters which are the same as in the reverse complementary second and first lists of characters, respectively;

(xxxv) adding up

- $C_c$; and
- the number of characters in the reverse complementary first list of characters which are located between the characters and/or longest continuous sequences of characters that were excluded from the reverse complementary first list of characters, and which were not excluded in any of the steps (xxix) to (xxxiii) of step (c); and
- the number of characters in the reverse complementary second list of characters which are located between the characters and/or longest continuous sequences of characters that were excluded from the reverse complementary second list of characters, and which were not excluded in any of the steps (xxix) to (xxxiii) of step (c),

to obtain the total number of characters, $C_t$, in the reverse complementary first and second lists of characters; and
(xxxvi) calculating DS according to the following formula:

$$DS_{rcs} = C_c / C_t$$

wherein when DS is determined for each first list of characters obtained in step (a) using sub-steps (i) to (x), $DS_{rcs}$ is determined for each corresponding reverse complementary first list of characters using sub-steps (xix) to (xxviii), and when DS is determined for each first list of characters obtained in step (a) using sub-steps (xi) to (xviii), $DS_{rcs}$ is determined for each corresponding reverse complementary first list of characters using sub-steps (xxix) to (xxxvi); and selecting, for each first list of characters obtained in step (a) and its corresponding reverse complementary first list of characters, the DS or $DS_{rcs}$ of highest value, $DS_{HV}$;

(e) adding up the number of first lists of characters which have a $DS_{HV}$ that is greater than a threshold value, T, to obtain the total number of first lists of characters, $L_c$, which are the same as a second list of characters;
(f) adding up

- $L_c$; and
- the number of first lists of characters which do not have a $DS_{HV}$ that is greater than T,

to obtain the total number of first lists of characters, $L_t$; and
(g) calculating the level of minimal residual disease (MRD) according to the following formula:

$$MRD = L_c \times (D / k) / L_t^2;$$

wherein in sub-steps (iii) and (xiii) of step (c), and sub-steps (xxi) and (xxxi) of step (d) of the present invention, selection is preferably repeated simultaneously for the continuous sequence of characters which is located to the left of the character or longest continuous sequence of characters excluded in sub-steps (ii) and (xii) of step (c), and sub-steps (xx) and (xxx) of step (d), respectively, and for the continuous sequence of characters which is located to the right of the character or longest continuous sequence of characters excluded in sub-steps (ii) and (xii), and sub-steps (xx) and (xxx) of step (d), respectively; and
wherein in sub-step (v) of step (c) and sub-step (xxiii) of step (d) of the present invention, selection is preferably repeated simultaneously for the continuous sequence of characters to the immediate left of each character or each longest continuous sequence of characters excluded in the previous step, and for the continuous sequence of characters to the immediate right of each character or each longest continuous sequence of characters excluded in the previous step.

**Brief description of the figures**

**Figure 1.** Schematic diagram representing sub-steps (i) to (vii) of step (c) according to the invention, wherein the narrow grey line ( ) represents a first character list and the narrow black line (-) represents a second character list. Selection of a character or longest continuous sequence of characters which are the same in the first list of characters and the second list of characters is represented by broader lines ( ) and ( ), respectively, that are subsequently excluded from the aforementioned character lists.

**Figure 2.** Schematic diagram representing sub-steps (xi) to (xv) of step (c) according to the invention, wherein the narrow grey line ( ) represents a first character list and the narrow black line (-) represents a second character list. Selection of a character or longest continuous sequence of characters which are the same in the first list of characters and the second list of characters is represented by broader lines ( ) and ( ), respectively, that are subsequently excluded from the aforementioned character lists.

**Figure 3.** Sensitivity of the method of the present invention in the characterization of gene rearrangements of immunoglobulins in samples from multiple myeloma patients. The percentage of clonotypic readings is measured by serial dilution of clonal rearrangements on a polyclonal background obtained from healthy patients or from non-B cell lines, wherein the sensitivity depends on the number of variants found in a clonotypic reading.

**Figure 4.** Correlation ($R^2$ = 0.51) between MRD data measured with flow cytometry (Log EMR FCM, y axis) and massive parallel sequencing (Log EMR NGS, x axis) for main rearrangements in follow-up samples of patients diagnosed with and treated for multiple myeloma.

**Figure 5.** Quantification of sensitivity of the method of the present invention in the characterization of point mutations (DNM3A, IDH2, FLT3 and NMP1) in acute myeloid leukemia (AML) patients. The percentage of clonotypic readings is measured in serial dilutions of AML mutated genes.

**Detailed description of the invention**

The present invention relates to a method for quantifying the level of disease. In particular, the present invention relates to a method for quantifying the level of minimal residual disease (MRD). MRD is the name given to the disease that remains in a subject after treatment of said disease. Thus, quantifying the level of MRD means quantifying the number of diseased cells in a subject or quantifying the amount of genetic material that is associated with disease in a subject after treatment of said disease. Preferably, quantifying the level of MRD means quantifying the number of diseased cells in a biological sample or tissue from a subject after treatment of said disease, or quantifying the level of MRD means quantifying the amount of genetic material that is associated with disease in a biological sample or tissue from a subject, after treatment of said disease. A diseased cell may be identified based on the expression or lack of expression of a biological marker on the diseased cell surface and/or inside said diseased cell, or based on the presence of at least one molecule foreign to the cell on the diseased cell surface and/or inside said diseased cell.

In the present invention, the disease is a genetic disease. Said genetic disease is characterised by at least one variant or the absence of said at least one variant in a nucleotide sequence, wherein said variant is preferably a clonotypic nucleotide sequence for immunoglobulin gene rearrangements, high allelic load, a point mutation (SNV), a multiple mutation (MNV), an indel, a long insertion, a long deletion and/or a translocation. More preferably said disease is characterised by high allelic load and/or at least one tumor clonotypic nucleotide sequence for at least one immunoglobulin gene rearrangement, at least one point mutation (SNV), at least one multiple mutation (MNV), at least one indel, at least one long insertion, at least one long deletion and/or at least one translocation. In one preferred embodiment of the invention, the disease is selected from cancer or leukaemia. In a further preferred embodiment of the invention, said disease is selected from acute lymphoblastic leukaemia, acute myeloid leukaemia, chronic lymphocytic leukaemia, chronic myelogenous (or myeloid) leukemia, follicular lymphoma, mantle cell lymphoma, multiple myeloma, breast cancer or neuroblastoma. Furthermore preferably, said disease is characterised by:

- a point mutation (SNV), multiple mutation (MNV), and/or indel selected from a FLT3 internal tandem duplication (FLT3-ITD) or a nucleophosmin1 (NMP1) mutation in acute myeloid leukaemia;
- a long insertion and/or translocation selected from t(9;22) BCR-Abl or t(12;21) ETV6-RUNX1 (TEL-AML1) in acute lymphoblastic leukaemia; t(15;17) PML-RARa, t(8;21) AML1-RUNX1T1 (AML-ETO) or inv(16) CBFb/MYH11 in acute myeloid leukaemia; t(9;22) BCR-Abl in chronic myeloid leukaemia, t(14;18) IgH/BCL2 in follicular lymphoma; t(11;14) IgH/CCND1 (IgH/ BCL1) in mantle cell lymphoma, or t(4;14) in multiple myeloma; or
- a patient-specific immunoglobulin rearrangement in acute lymphoblastic leukaemia, multiple myeloma, mantle cell lymphoma, follicular lymphoma, chronic lymphocytic leukaemia or acute lymphoblastic leukaemia. Further, the method of the present invention may be applied to any of the haematological neoplasias disclosed in Hauwel M., Matthes T. "Minimal residual disease monitoring: the new standard for treatment evaluation of haematological malignancies?"; Swiss Med Wkly. (2014) 144:w13907].

In an alternative embodiment, the method of the invention is therefore a method for quantifying the level of allelic load and/or at least one clonotypic nucleotide sequence for at least one immunoglobulin gene rearrangement, at least one point mutation (SNV), at least one multiple mutation (MNV), at least one indel, at least one long insertion, at least one long deletion and/or at least one translocation in a subject who has been treated for a disease.

In the present invention, the level of MRD is quantified in a subject who has been treated for said disease by a method comprising seven steps, (a) to (g). The step (a) comprises the sequential steps of:

- amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- sequencing said at least one nucleotide sequence to obtain at least one first list of characters reading from left to right.

In an analogous manner, the step (b) comprises the sequential steps of:

- amplifying by polymerase chain reaction using the same primers as in step (a), at least one nucleotide sequence in a biological sample obtained from said subject prior to treatment for said disease; and
- sequencing said at least one nucleotide sequence to obtain at least one second list of characters reading from left to right. Step (a) may be performed simultaneously with step (b), or step (a) may be performed before or after step (b). Preferably, step (b) is performed before step (a).

The biological sample in steps (a) and (b) comprises a sample of biological matter taken from a subject. Said biological sample comprises at least one nucleotide sequence in at least one cell. Preferably said biological sample comprises at least one nucleotide sequence in the genomic DNA of at least one cell in a tissue, blood, urine, faeces, saliva, mucus, sperm, bone, hair and/or nails. The biological sample in step (a) is a test (or follow-up) sample diagnostic for minimal residual disease.The biological sample in step (b) is a diagnosis (or calibration or control) sample diagnostic for the disease prior to a treatment. Preferably, the biological sample in step (b) is a sample with high allelic or clonal load which was taken before treatment of the disease from the same subject as the biological sample in step (a). The biological sample in step (a) has an average weight, k, of genomic DNA per diploid cell.

Each nucleotide sequence in the genomic DNA is amplified by PCR using primers, whereby said primers comprise a forward primer and a reverse primer which bind to different complementary sequences on the Watson and Crick strands adjacent to said nucleotide sequence, thereby identifying the 5' and 3' limits of said nucleotide sequence. In particular, the 3' end of the nucleotide sequence of the Watson strand begins with the nucleotide which is adjacent to the nucleotide at the 5' end of the sequence that is annealed with the forward primer. Conversely, the 5' end of the nucleotide sequence of the Watson strand begins with the nucleotide complementary to the nucleotide which is adjacent to the nucleotide at the 5' end of the sequence that is annealed with the reverse primer. Likewise, the 3' end of the nucleotide sequence of the Crick strand begins with the nucleotide which is adjacent to the nucleotide at the 5' end of the sequence that is annealed with the reverse primer. Conversely, the 5' end of the nucleotide sequence of the Crick strand begins with the nucleotide complementary to the nucleotide which is adjacent to the nucleotide at the 5' end of the sequence that is annealed with the forward primer. Accordingly, a DNA polymerase attaches to the 5' end of the aforementioned primers and replicates the nucleotide sequence multiple times.

Preferably the primers are locus-specific primers chosen so as to identify at least one specific variant of a nucleotide sequence present in the biological sample in step (b), wherein said at least one variant or the absence of said at least one variant is indicative of disease. In particular, said at least one variant comprises at least one clonotypic nucleotide sequence for at least one immunoglobulin gene rearrangement, at least one point mutation (SNV), at least one multiple mutation (MNV), at least one indel, at least one long insertion and/or at least one translocation. The at least one specific variant of a nucleotide sequence present in the biological sample obtained from the subject prior to treatment for the disease is the at least one specific variant of a nucleotide sequence which is indicative of said disease and identified in said biological sample in greatest proportion. Thus, each specific variant indicative of said disease is identified and the proportion of each specific variant is compared, with the specific variant or specific variants which is or are ranked in greatest proportion respectively being that or those which locus-specific primers are chosen to identify. It should be noted that in one embodiment the process of ranking is a step which is carried out by the FrequencyRank.java script.

As a consequence of the fact that at least one specific variant of a nucleotide sequence is thus identified in steps (a) and (b), and/or more than one type of primer may be used in steps (a) and (b), the steps (a) and (b) involves identifying, amplifying and sequencing at least one nucleotide sequence (*i.e.* one or more nucleotide sequences) in a biological sample, thus affording at least one list of characters (*i.e.* one or more lists of characters) corresponding thereto. However, in a more preferred embodiment, the primers used in steps (a) and (b) are a locus-specific forward primer and locus specific reverse primer chosen so as to identify one specific variant of a nucleotide sequence present in the biological samples, wherein said one variant or the absence of said one variant is indicative of disease. Thus, in this preferred embodiment, the step (b) comprises the sequential steps of:

- amplifying by polymerase chain reaction using the same locus-specific forward primer and locus specific reverse primer used in step (a), a nucleotide sequence in a biological sample obtained from said subject prior to treatment for said disease of said nucleotide sequence; and
- sequencing said nucleotide sequence to obtain a second list of characters reading from left to right.

Thus, amplification of at least one nucleotide sequence present in each biological sample is performed with specific primers identifying at least one region of interest (*i.e.* at least one specific variant indicative of the disease for which a subject has been treated), before processing each on a massively parallel sequencing platform. Accordingly, the test sample on this at least one region of interest was amplified and sequenced with higher, or equal to, expected sensitivity coverage. For amplification of the test sample, an amount, D, of genomic DNA (gDNA) from said test (follow-up) sample is used in PCR, and amplification is preferably repeated until a quantity sufficient for sequencing with a desired sensitivity is obtained. Preferably, an amount, D, of gDNA from said test sample is used in PCR to ensure that a sensitivity equivalent to that obtainable from sampling a given number of cells is obtained. The sensitivity is determined in every instance for application to the study of disease and residual circulating tumor cells.

The amount, D, of the genomic DNA from the test (follow-up) sample which is used in PCR for sequencing with a desired sensitivity (S) is established first by measuring the concentration of DNA ([DNA], pg/μL) in the biological sample obtained from a subject after treatment for said disease (test sample). This value is then used to determine the number of equivalent cells per microliter (N) of the test sample according to the following formula:

$$N = [DNA] / k$$

wherein N and [DNA] are as defined above, and k is the average weight of the genomic DNA per diploid cell of the test sample, whereby k preferably assumes a value of 6.49 picograms per cell. The number of equivalent cells per microliter (N) of the test sample subsequently allows calculation of the volume of sample (V, μL) which it is necessary to use in the PCR in order to reach a desired sensitivity (S) according to the following formula:

$$V = 1 / (N \times S)$$

A sensitivity of $10^{-5}$ equates with that achievable from use of genomic DNA from at least 100,000 equivalent cells. The volume of test sample (V) determines the number of PCR experiments necessary to obtain a sufficient amount (D, pg) of genomic DNA for sequencing and, in addition, is used to calculate the amount (D) of the genomic DNA from the test sample which is used in PCR according to the following formula:

$$D = [DNA] \times V$$

Amplification may be performed by any one of the following PCR techniques selected from multiplex-PCR, and single PCR using a pair of primers. Preferably amplification is performed by multiplex-PCR.

Optionally, steps (a) and (b) may comprise a further step of isolating said at least one amplified nucleotide sequence prior to the step of sequencing using routine methods in the art. Thus, the first step of steps (a) and (b) comprises amplification of at least one nucleotide sequence obtained from at least one longer nucleotide sequence by selective amplification of said at least one nucleotide sequence over said at least one longer nucleotide sequence, wherein each longer nucleotide sequence comprises a polynucleotide, wherein said polynucleotide is preferably selected from double- or single-stranded DNA or RNA, more preferably double-stranded DNA, furthermore preferably double stranded genomic DNA. When said polynucleotide is single-stranded DNA, a complementary sequence is synthesised therefrom prior to carrying out steps (a) or (b) to afford double-stranded DNA. When said polynucleotide is RNA, a complementary double-stranded DNA is synthesised (retrotranscribed) therefrom prior to carrying out steps (a) or (b).

The at least one nucleotide sequence of each of steps (a) and (b), thus amplified and optionally isolated, is subsequently sequenced. Sequencing of a nucleotide sequence of step (a) affords a first list of characters reading from left to right corresponding thereto, wherein each first list of characters has a total number of characters, $C_t$. Moreover, the total number of first lists of characters ($L_t$) corresponds to the total number of different nucleotide sequences in step (a). Sequencing of a nucleotide sequence of step (b) likewise affords a second list of characters reading from left to right, corresponding thereto.

The sequencing is a multiplex and/or high-throughput nucleotide sequencing technique. Preferably, the sequencing is performed by a next-generation technique, more preferably massively parallel sequencing [*e.g.* massively parallel signature sequencing (MPSS)]. In one embodiment of the present invention, when multiple primers are used in sequencing, the sequencing steps in steps (a) and (b) are performed using barcodes to identify between the different primers used. In one especially preferred embodiment of the present invention, the sequencing is performed by massively parallel sequencing using emulsion-PCR.

Each of the separate steps of amplifying and sequencing said at least one nucleotide sequence in steps (a) and (b) may be performed by separate means (*i.e.* by separate instruments). Alternatively, two or all of these separate steps may be performed by the same instrument.

Sequencing of a nucleotide sequence in steps (a) and (b) affords a corresponding list of characters, whereby each character in each list of characters comprises a letter. In one embodiment of the present invention, sequencing of a nucleotide sequence in steps (a) and (b) affords a corresponding list of characters, whereby each character in each list of characters comprises a letter associated with a number (or symbol). More preferably, each letter represents the nucleotide that is identified at the corresponding position in the nucleotide sequence which has the highest quality (*Q*) within the limits of the sequencing method, and the number or symbol associated therewith is the quality (*Q*), wherein *Q* is an integer mapping of the probability that the letter which represents a nucleotide that is identified at the corresponding position in the nucleotide sequence is incorrect. Thus, each of the lists of characters obtained in steps (a) and (b) of the present invention is preferably comprised in a sequence format file, more preferably a FASTQ file.

Alternatively, each character more preferably represents the nucleotide that is identified at the corresponding position in the nucleotide sequence in greatest proportion. In one furthermore preferred embodiment of this alternative, the letter associated with said character represents the nucleotide that is identified at the corresponding position in the nucleotide sequence in highest proportion and the number or symbol associated therewith is the proportion (*e.g.* as a percentage, fraction or ratio) of said nucleotide that is identified therein.

A continuous sequence of characters is a list which is unbroken by another character or absence of a character, wherein said continuous sequence of characters represents an unbroken continuous sequence of nucleotides. Analogous with that described above, each character in the continuous sequence of characters comprises one or more letter, preferably one or more letter associated with a number or symbol, more preferably wherein each letter represents the nucleotide that is identified at the corresponding position in the nucleotide sequence which has the highest quality (*Q*) within the limits of the sequencing method, and the number or symbol associated therewith is the quality (*Q*), wherein *Q* is an integer mapping of the probability that the letter which represents a nucleotide that is identified at the corresponding position in the nucleotide sequence is incorrect. As such, in this more preferred embodiment of the invention, said continuous sequence of characters comprises a continuous sequence of letters representing a continuous sequence of nucleotides, when each character in the continuous sequence of characters represents the nucleotide that is identified at the corresponding position which has the highest quality (*Q*) within the limits of the sequencing method.

Alternatively, each character in the continuous sequence of characters preferably comprises a letter associated with a number or symbol, more preferably wherein each letter represents the nucleotide that is identified at the corresponding position in the nucleotide sequence in greatest proportion. In one embodiment of this more preferred alternative, the letter associated with said character in the continuous sequence of characters represents the nucleotide that is identified at the corresponding position in the nucleotide sequence in highest proportion and the number or symbol associated therewith is the proportion (*e.g*. as a percentage, fraction or ratio) of said nucleotide that is identified therein. As such, in this more preferred alternative embodiment, said continuous sequence of characters comprises a continuous sequence of letters representing a continuous sequence of nucleotides, when each character in the continuous sequence of characters represents the nucleotide that is identified at the corresponding position in the continuous sequence of nucleotides in greatest proportion.

Each character in each list of characters corresponds to one nucleotide in said nucleotide sequence and the order of characters in said list corresponds to the order of nucleotides in said nucleotide sequence. Thus, the character at the left-hand end of said list corresponds to the nucleotide or proportion of nucleotides at the 3' end of the Watson strand of said nucleotide sequence and the character at the right-hand end of said list corresponds to the nucleotide or proportion of nucleotides at the 5' end of the Watson strand of said nucleotide sequence. Analogously, a complementary (or partly complementary) list of characters is obtained representing each Crick strand of said nucleotide sequence, whereby the character at the left-hand end of said list corresponds to the nucleotide or proportion of nucleotides at the 3' end of the Crick strand of said nucleotide sequence and the character at the right-hand end of said list corresponds to the nucleotide or proportion of nucleotides at the 5' end of the Crick strand of said nucleotide sequence.

Subsequently, comparison of each first list of characters obtained in step (a) is made with each second list of characters obtained in step (b). Said comparison is made so as to ultimately determine the total number of first lists of characters, $L_c$, which are the same as a second list of characters. In other words, the comparison is made so as to determine the $L_c$ which are identical with (*i.e.* match) a second list of characters. In order to determine $L_c$, it is necessary to determine the degree of similarity of each first list of characters obtained in step (a) with each second list of characters obtained in step (b), wherein a degree of similarity, DS, is determined for a first list of characters obtained in step (a) with a second list of characters obtained in step (b). Although methods adapted to bioinformatics are known which access external data (e.g. genetic databases derived from populations) in order to carry out the comparison step and somehow implement "biological knowledge" the method of the present invention works without the need to access external data. To this end, the first feature that is considered essential to implement is a fuzzy logic. The rate of failure of sequencers using a classical binary logic - in which sequences can only be equal or different - is so high that it is not useful. A high proportion (nearly all) of nucleotide sequences that evaluate as different, are equal but appear as different because of an error in the sequencer. Therefore, a comparison process to evaluate the degree of similarity between any two lists of characters is implemented.

In one embodiment of the invention, each character in a list of characters comprises a letter, such that a character in the first list of characters is determined as the same as a character in the second list of characters, when the letter is the same in the first and second lists of characters (*i.e.* a character in one list of characters is determined as the same as a character in another list of characters when the letters are the same in each list). In one preferred embodiment of the invention, each character in a list of characters comprises a letter associated with a number or symbol, more preferably wherein each letter represents the nucleotide that is identified at the corresponding position in the nucleotide sequence in the highest quality (*Q*) within the limits of the sequencing method and wherein each number or symbol represents the quality (*Q*). Thus, in said more preferred embodiment of the method of the invention, wherein each character in a first list of characters and each character in a second list of characters comprises a letter associated with a number or symbol, wherein said number or symbol represents quality (*Q*) and wherein said letter represents the nucleotide that is identified at the corresponding position in the nucleotide sequence having the highest quality (*Q*), a character in the first list of characters is determined as the same as a character in the second list of characters, when the letter having the highest quality is the same in the first and second lists of characters (*i.e.* a character in one list of characters is determined as the same as a character in another list of characters when the letters are the same in each list). Further to this, not only the letters but also the numbers or symbols associated therewith may be compared between lists, preferably by comparing the letter and the number or symbol representing the quality (*Q*) associated therewith for each character in each list. Thus, in a yet more preferred embodiment of the method of the invention, a character in one list of characters which comprises a letter associated with a quality (*Q*) is determined as the same as a character in another list of characters which comprises a letter associated with a quality (*Q'*), when the letter having the highest quality is the same in each list, and the quality (*Q*) of letters is the same in each list within a cut-off limit or an error, more preferably a cut-off limit. For example, a character at a given position which is assigned as T with a quality of 1.00 (*i.e.* 100%) may be considered the same as a character at a given position which is assigned as T with a quality of 0.99 (*i.e.* 99%), when the cut-off limit is set at 0.99 (*i.e.* the error is set at 1%). Thus, in the following step (c), each step of selecting the character or longest continuous sequence of characters which are the same, within a cut-off limit, in the first and second lists of characters or parts thereof, comprises firstly making the aforementioned comparison between the first list of characters and the second list of characters or parts thereof, and secondly choosing the character or longest continuous sequence of characters based on the criteria given in the following, when one or more characters or one or more continuous sequences of characters are identified as longest from said comparison. In this method, the cut-off limit is preferably set at a quality (*Q*) of 0.99, more preferably at 0.999, furthermore preferably at 0.9999, most preferably 0.99999, and/or the error is set at a maximum of 1%, more preferably 0.1%, furthermore preferably 0.01%, most preferably 0.001%. In one yet more preferred embodiment of the method of the invention, a character in a first list of characters is determined as the same as a character in a second list of characters, when the letter having the highest quality (*Q*) is the same in the first and second lists of characters, and the quality of the letter in the first list of characters is within 0.01 (1%) of the quality of the letter in the second list of characters, furthermore preferably within 0.001 (0.1%), still more preferably within 0.0001 (0.01%), most preferably within 0.00001 (0.001%).

Alternatively, comparison is performed by comparing the letter comprising each character which is present in greatest quality (*Q*) or in greatest proportion in each list of characters. Thus, in the method of the invention, a character in one list of characters is determined as the same as a character in another list of characters preferably when the letters are the same.

Alternatively, comparison is performed by comparing the proportion of each one or more letter comprising each character. Thus, comparison is performed by comparing the proportion of each one or more nucleotide that is identified at each position in the nucleotide sequence. In this method, a character in one list of characters is determined as the same as a character in another list of characters when the proportion of letters is the same within error. For example, a character for which the proportion of A at a given position is 0.11 and the proportion of T at said given position is 0.89 (*i.e.* the ratio of A:T is 0.11:0.89) may be considered the same as a character for which the proportion of A at a given position is 0.1 and the proportion of T at said given position is 0.9 (*i.e.* the ratio of A:T is 0.1:0.9), when the error is set at 5% error. Thus, in the following step (c), each step of selecting the character or longest continuous sequence of characters which are the same in the first list of characters and second list of characters or parts thereof comprises firstly making the aforementioned comparison between the first list of characters and second list of characters or parts thereof, and secondly choosing the character or longest continuous sequence of characters based on the criteria given in the following, when one or more characters or one or more continuous sequences of characters are identified as longest from said comparison. In this method, the error is set at a maximum of 1%, more preferably 0.1%, furthermore preferably 0.01%, most preferably 0.001%.

Thus, for each first list of characters obtained in step (a), the degree of similarity with each second list of characters obtained in step (b) is subsequently determined in step (c), wherein a degree of similarity, DS, of a first list of characters obtained in step (a) with a second list of characters obtained in step (b) is determined either by sub-steps (i) to (x) [sub-steps (i) to (vii) of which are represented schematically in **Figure 1**] or (xi) to (xviii) [sub-steps (xi) to (xv) of which are represented schematically in **Figure 2**]. In particular, a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and
(xviii) calculating DS according to the following formula:

$$DS = C_c / C_t.$$

In step (c), the sub-steps (i) and (xi) of selecting the character which is the same in the first and second lists of characters involve comparing individual characters in the first and second lists of characters according to the foregoing criteria for comparison. Moreover, the sub-steps (i) and (xi) of selecting the longest continuous sequence of characters which is the same in the first and second lists of characters involve comparing consecutive individual characters in the first and second lists of characters according to the foregoing criteria for comparison. It should be noted that sub-steps (i) to (iv) and (xi) to (xiv) are identical.

In step (c), after each step of selecting the character or longest continuous sequence of characters which are the same in the first and second lists of characters or parts thereof, a step of excluding said character or longest continuous sequence of characters, thus selected, takes place, wherein each step of excluding comprises removing the character or longest continuous sequence of characters, thus selected, from consideration in subsequent steps of selecting the character or longest continuous sequence of characters which is the same in the first and second lists of characters. It should be noted that each step of excluding results in a non-continuous sequence of characters which is broken at the point between each character which flanks the character or longest continuous sequence of characters, thus excluded. As such, any subsequent step of selecting the character or longest continuous sequence of characters which are the same in the first and second lists of characters or parts thereof in sub-steps (v) to (vii) of step (c) does not consider a sequence which extends beyond a previously excluded character or longest continuous sequence of characters, but instead considers the continuous sequence of characters located adjacent to each character or each longest continuous sequence of characters excluded in the previous step. Moreover, any subsequent step of selecting the character or longest continuous sequence of characters which are the same in the first and second lists of characters or parts thereof in sub-steps (xiii) to (xv) will not consider a sequence which bridges the characters on either side of the excluded character or longest continuous sequence of characters.

Each cycle of selecting and excluding a character or longest continuous sequence of characters which is the same in the first and second lists of characters is repeated until no character or longest continuous sequence of characters which is the same in the first and second lists of characters is selected. In sub-steps (iii) and (xiii) of step (c) of the present invention, selection is preferably repeated simultaneously for the continuous sequence of characters which is located to the left of the character or longest continuous sequence of characters excluded in sub-steps (ii) and (xii), respectively, and for the continuous sequence of characters which is located to the right of the character or longest continuous sequence of characters excluded in sub-steps (ii) and (xii), respectively. Alternatively, this may be repeated first for the continuous sequence of characters which is located to the left of the character or longest continuous sequence of characters excluded in sub-steps (ii) and (xii), respectively, and then for the continuous sequence of characters which is located to the right of the character or longest continuous sequence of characters excluded in sub-steps (ii) and (xii), respectively. Alternatively, this may be repeated first for the continuous sequence of characters which is located to the right of the character or longest continuous sequence of characters excluded in sub-steps (ii) and (xii), respectively, and then for the continuous sequence of characters which is located to the left of the character or longest continuous sequence of characters excluded in sub-steps (ii) and (xii), respectively.

Analogously, in sub-step (v) of step (c) of the present invention, selection is preferably repeated simultaneously for the continuous sequence of characters to the immediate left of each character or each longest continuous sequence of characters excluded in the previous step, and for the continuous sequence of characters to the immediate right of each character or each longest continuous sequence of characters excluded in the previous step. Alternatively, this may be repeated first for the continuous sequence of characters to the immediate left of each character or each longest continuous sequence of characters excluded in the previous step, and then for the continuous sequence of characters to the immediate right of each character or each longest continuous sequence of characters excluded in the previous step. Alternatively, this may be repeated first for the continuous sequence of characters to the immediate right of each character or each longest continuous sequence of characters excluded in the previous step, and then for the continuous sequence of characters to the immediate left of each character or each longest continuous sequence of characters excluded in the previous step.

Each of these approaches is novel compared to standard implementations and generates a marginally more compact comparison result.

Moreover, in a preferred embodiment of step (c), each step of selecting the character or longest continuous sequence of characters is a step of selecting the longest continuous sequence of characters, wherein said longest continuous sequence of characters comprises a minimum of two characters. As such, each cycle of selecting and excluding a character or longest continuous sequence of characters which is the same in the first and second lists of characters is repeated in step (c) until no longest continuous sequence of characters having a minimum of two characters which is the same in the first and second lists of characters is selected. More preferably, said longest continuous sequence of characters comprises a minimum of 3 characters, furthermore preferably a minimum of 4 characters.

Once it is not possible to select a character or longest continuous sequence of characters which is the same in the first list of characters and second list of characters (because all characters or longest continuous sequences of characters which are the same in the first list of characters and second list of characters have been excluded), the total number of characters, $C_c$, which were excluded in the first list of characters and excluded in the second list of characters is obtained by sub-steps (viii) and/or (xvi) of step (c), wherein the number of characters in the first list of characters which were respectively excluded in any of the sub-steps (i) to (vii) and (xi) to (xv) and the number of characters in the second list of characters which were respectively excluded in any of the sub-steps (i) to (vii) and (xi) to (xv) is added up. The total number of characters, $C_c$, which were excluded in the first and second lists of characters may also be thought of as 2 x (the number of characters which were excluded in the first list of characters), or as 2 x (the number of characters which were excluded in the second list of characters). Analogously, the total number of characters, $C_t$, in the first list of characters is obtained by sub-steps (ix) and/or (xvii) of step (c), wherein $C_c$, plus the number of characters in the first list of characters which are located between the characters and/or longest continuous sequences of characters that were excluded from the first list of characters which were not excluded in any of the sub-steps (i) to (vii) and (xi) to (xv) of step (c), plus the number of characters in the second list of characters which are located between the characters and/or longest continuous sequences of characters that were excluded from the second list of characters which were not excluded in any of the sub-steps (i) to (vii) and (xi) to (xv) of step (c), is added up.

Thus, the method of the present invention is intended to detect a list of characters representing a specific nucleotide sequence, which is supplied as an argument to the method, within a data file that contains a mix of lists of characters each representing a nucleotide sequence fragmented in places that, from an informatics point of view are considered random. Therefore, the at least one lists of characters in the mix have random lengths and it is not known in advance where a list of characters representing a specific nucleotide sequence [in step (b)] can be found in each at least one list of characters. Thus, the method of the present invention comprises a combination of alignment and comparison. Since it comprises a mix of alignment and comparison, it is considered that in the invention, comparison is made only from the first character or longest continuous sequence of characters which is the same in the first list of characters and the second list of characters and the last character or longest continuous sequence of characters which is the same in the first and second lists of characters *(i.e.* including and between the matching characters or longest continuous sequences of characters closest to the extremes of the first and second lists of characters), whereby $C_c$ and $C_t$ are determined over that portion of the first and second lists of characters which is from the first character or longest continuous sequence of characters which is the same in the first and second lists of characters and the last character or longest continuous sequence of characters which is the same in the first and second lists of characters. Accordingly, in a preferred embodiment, each of the characters or longest continuous sequences of characters which are eliminated in either of sub-steps (i) to (vii) or (xi) to (xv) of step (c) are placed in a .dna file and sub-steps (viii) and (xvi) may use the data in a .dna file to calculate $C_c$ using a -trim option, such that comparison is made only between the first and the last character or longest continuous sequence of characters in the first list which are the same as in the second list.

Subsequent to step (c), a step (d) is carried out, in which for each first list of characters obtained in step (a), the DS of highest value, $DS_{HV}$, is selected. However, by virtue of the fact that each nucleotide sequence in steps (a) and (b) (arbitrarily herein defined as the Watson strand) has a complementary nucleotide sequence (in particular the reverse complementary sequence, arbitrarily herein defined as the Crick strand), such that first list of characters obtained in step (a) and a second list of characters obtained in step (b) also have a corresponding reverse complementary first list of characters and a corresponding reverse complementary second list of characters, respectively, in a preferred embodiment, not only the aforementioned nucleotide sequence in steps (a) and (b) but also the complementary nucleotide sequence may be subjected to steps (a), (b) and (c) of the method of the invention. Thus, in said preferred embodiment, the step (d) comprises:

- amplifying by polymerase chain reaction using primers, each at least one nucleotide sequence which is the reverse complementary sequence complementary to the at least one nucleotide sequence in step (a), and sequencing said at least one reverse complementary nucleotide sequence to obtain at least one reverse complementary first list of characters reading from left to right; and
- amplifying by polymerase chain reaction using the same primers as in the previous step, each at least one nucleotide sequence which is the reverse complementary sequence complementary to the at least one nucleotide sequence in step (b), and sequencing said at least one reverse complementary nucleotide sequence to obtain at least one reverse complementary second list of characters reading from left to right; and
- determining, for each reverse complementary first list of characters obtained in step (a), the degree of similarity with each reverse complementary second list of characters obtained in step (b), wherein a degree of similarity, $DS_{rcs}$, of a reverse complementary first list of characters obtained in step (a) with a reverse complementary second list of characters obtained in step (b) is determined either by:

    (xix) selecting the character or longest continuous sequence of characters which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected;
    (xx) excluding the character or longest continuous sequence of characters selected in step (xix) from subsequent steps of selecting a character or longest continuous sequence of characters which is the same in the reverse complementary first list of characters and the reverse complementary second list of characters;
    (xxi)

    - selecting the character or longest continuous sequence of characters which is located to the left of the character or longest continuous sequence of characters excluded in step (xx) which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
    - selecting the character or longest continuous sequence of characters which is located to the right of the character or longest continuous sequence of characters excluded in step (xx) which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxii) excluding each character and/or each longest continuous sequence of characters selected in step (xxi) from subsequent steps of selecting a character or longest continuous sequence of characters which is the same in the reverse complementary first list of characters and the reverse complementary second list of characters;
(xxiii)

- selecting the character or longest continuous sequence of characters which is located in the continuous sequence of characters to the immediate left of each character or each longest continuous sequence of characters excluded in the previous step which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
- selecting the character or longest continuous sequence of characters which is located in the continuous sequence of characters to the immediate right of each character or each longest continuous sequence of characters excluded in the previous step which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxiv) excluding each character and/or each longest continuous sequences of characters selected in step (xxiii) from subsequent steps of selecting a character or longest continuous sequence of characters which is the same in the reverse complementary first list of characters and the reverse complementary second list of characters;
(xxv) repeating steps (xxiii) and (xxiv) until no character or longest continuous sequence of characters which is the same in the reverse complementary first list of characters and the reverse complementary second list of characters is selected;
(xxvi) adding up

- the number of characters in the reverse complementary first list of characters which were excluded in any of the steps (xix) to (xxv); and
- the number of characters in the reverse complementary second list of characters which were excluded in any of the steps (xix) to (xxv) to obtain the total number of characters, $C_c$, in the reverse complementary first and second lists of characters which are the same as in the reverse complementary second and first lists of characters, respectively;

(xxvii) adding up

- $C_c$; and
- the number of characters in the reverse complementary first list of characters which are located between the characters and/or longest continuous sequences of characters that were excluded from the reverse complementary first list of characters, and which were not excluded in any of the steps (xix) to (xxv) of step (c); and
- the number of characters in the reverse complementary second list of characters which are located between the characters and/or longest continuous sequences of characters that were excluded from the reverse complementary second list of characters, and which were not excluded in any of the steps (xix) to (xxv) of step (c),

to obtain the total number of characters, $C_t$, in the reverse complementary first and second lists of characters; and
(xxviii) calculating DS according to the following formula:

$$DS_{rcs} = C_c / C_t$$

or by:

(xxix) selecting the character or longest continuous sequence of characters which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected;
(xxx) excluding the character or longest continuous sequence of characters selected in step (xxix) from subsequent steps of selecting a character or longest continuous sequence of characters which is the same in the reverse complementary first list of characters and the reverse complementary second list of characters;
(xxxi)

- selecting the character or longest continuous sequence of characters which is located to the left of the character or longest continuous sequence of characters excluded in step (xxx) which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
- selecting the character or longest continuous sequence of characters which is located to the right of the character or longest continuous sequence of characters excluded in step (xxx) which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxxii) excluding each character and/or each longest continuous sequence of characters selected in step (xxxi) from subsequent steps of selecting a character or longest continuous sequence of characters which is the same in the reverse complementary first list of characters and the reverse complementary second list of characters;
(xxxiii) repeating steps (xxxi) and (xxxii) until no character or longest continuous sequence of characters which is the same in the reverse complementary first list of characters and the reverse complementary second list of characters is selected;
(xxvi) adding up

- the number of characters in the reverse complementary first list of characters which were excluded in any of the steps (xix) to (xxv); and
- the number of characters in the reverse complementary second list of characters which were excluded in any of the steps (xix) to (xxv) to obtain the total number of characters, $C_c$, in the reverse complementary first and second lists of characters which are the same as in the reverse complementary second and first lists of characters, respectively;

(xxvii) adding up

- $C_c$; and
- the number of characters in the reverse complementary first list of characters which are located between the characters and/or longest continuous sequences of characters that were excluded from the reverse complementary first list of characters, and which were not excluded in any of the steps (xix) to (xxv) of step (c); and
- the number of characters in the reverse complementary second list of characters which are located between the characters and/or longest continuous sequences of characters that were excluded from the reverse complementary second list of characters, and which were not excluded in any of the steps (xix) to (xxv) of step (c),

to obtain the total number of characters, $C_t$, in the reverse complementary first and second lists of characters; and
(xxviii) calculating DS according to the following formula:

$$DS_{rcs} = C_c / C_t$$

wherein when DS is determined for each first list of characters obtained in step (a) using sub-steps (i) to (x), $DS_{rcs}$ is determined for each corresponding reverse complementary first list of characters using sub-steps (xix) to (xxviii), and when DS is determined for each first list of characters obtained in step (a) using sub-steps (xi) to (xviii), $DS_{rcs}$ is determined for each corresponding reverse complementary first list of characters using sub-steps (xxix) to (xxxvi); and

selecting, for each first list of characters obtained in step (a) and its corresponding reverse complementary first list of characters, the DS or $DS_{rcs}$ of highest value, $DS_{HV}$.

Thus, a DS or $DS_{rcs}$ of 0.0 means that no characters in a first list of characters are the same as in a second list of characters, whereas a DS or $DS_{rcs}$ of 1.0 means that all characters in a first list of characters are the same as in a second list of characters *(i.e.* a nucleotide sequence from a biological sample from a subject after treatment for said disease is strictly equal with a nucleotide sequence from a biological sample obtained from a subject prior to treatment for said disease). Therefore, the method of the present invention provides information on how many nucleotide sequences in the biological sample from a subject contain the argument sequence (the nucleotide sequence from a biological sample obtained from a subject with said disease), either in its original (Watson) form or in its reverse complement (Crick) version.

Having determined $DS_{HV}$ for each of the at least one first list of characters obtained in step (a), the number of first lists of characters obtained in step (a) which have a $DS_{HV}$ that is greater than a threshold value, T, is subsequently added up in a step (e) to obtain the total number of first lists of characters, $L_c$, which are the same as a second list of characters. Similarly, in a step (f), $L_c$ and the number of first lists of characters which do not have a $DS_{HV}$ that is greater than T are added up to obtain $L_t$. $L_t$ corresponds to the total number of first lists of characters. Preferably said threshold value, T, for the DS and $DS_{rcs}$ is set at 0.99, more preferably at 0.999, furthermore preferably at 0.9999, most preferably at 0.99999. Steps (e) and (f) may be performed simultaneously or step (e) may be performed before or after step (f), preferably step (f) is performed after step (e).

Finally, a step (g) is performed to calculate the level of MRD. Calculating the level of MRD is performed according to the following formula:

$$MRD = L_c \text{ x } (D / k) / L_t^2$$

wherein $L_c$, D, k and $L_t$ are as previously defined, and as defined below:

$L_c$ = total number of first lists of characters which are the same as a second list of characters;
D = amount, D, of genomic DNA from a biological sample obtained from a subject after treatment for a disease (from which said at least one first list of characters is obtained by sequencing);
k = average weight, k, of genomic DNA per diploid cell from a biological sample obtained from a subject after treatment for a disease;
$L_t$ = total number of first lists of characters.

In one embodiment of the invention, an MRD of 1 is 100% indicative of disease in said subject and an MRD of 0 is 0% indicative of disease in said subject. In other words, an MRD of 1 indicates that said subject is suffering from said disease, whereas an MRD of 0 indicates that the subject is free of any disease (at least in the cells or tissue of the biological sample). As such, the MRD may be used to diagnose the presence of said disease in said subject and/or to determine the best therapeutic approach (if needed).

In one embodiment, the MRD is produced as an output (preferably in the form of a file, or on a screen or piece of paper) after step (d) has been performed. Preferably, the MRD, together with at least one first list of characters is produced as an output (preferably in the form of a file, or on a screen or piece of paper) detailing the character or longest continuous sequence of characters which is the same in the first and second lists of characters. Optionally, said output details the $C_c$, $C_t$, DS, $DS_{HV}$, D, k, $L_c$ and/or $L_t$, where relevant with the $DS_{rcs}$.

In an especially preferred embodiment, the present invention relates to a method for quantifying the level of minimal residual disease (MRD) in a subject who has been treated for said disease, which comprises:

(a)

- amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- sequencing said at least one nucleotide sequence to obtain at least one first list of characters reading from left to right;

(b)

- amplifying by polymerase chain reaction using the same primers as in step (a), at least one nucleotide sequence in a biological sample obtained from said subject prior to treatment for said disease; and
- sequencing said at least one nucleotide sequence to obtain at least one second list of characters reading from left to right;

(c) determining, for each first list of characters obtained in step (a), the degree of similarity with each second list of characters obtained in step (b), wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and
(xviii) calculating DS according to the following formula:

$$DS = C_c / C_t$$

(d)

- amplifying by polymerase chain reaction using primers, each at least one nucleotide sequence which is the reverse complementary sequence complementary to the at least one nucleotide sequence in step (a), and sequencing said at least one reverse complementary nucleotide sequence to obtain at least one reverse complementary first list of characters reading from left to right; and

  - amplifying by polymerase chain reaction using the same primers as in the previous step, each at least one nucleotide sequence which is the reverse complementary sequence complementary to the at least one nucleotide sequence in step (b), and sequencing said at least one reverse complementary nucleotide sequence to obtain at least one reverse complementary second list of characters reading from left to right; and

- determining, for each reverse complementary first list of characters obtained in step (a), the degree of similarity with each reverse complementary second list of characters obtained in step (b), wherein a degree of similarity, $DS_{rcs}$, of a reverse complementary first list of characters obtained in step (a) with a reverse complementary second list of characters obtained in step (b) is determined either by:

  (xix) selecting the character or longest continuous sequence of characters which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected;
  (xx) excluding the character or longest continuous sequence of characters selected in step (xix) from subsequent steps of selecting a character or longest continuous sequence of characters which is the same in the reverse complementary first list of characters and the reverse complementary second list of characters;
  (xxi)

  - selecting the character or longest continuous sequence of characters which is located to the left of the character or longest continuous sequence of characters excluded in step (xx) which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
  - selecting the character or longest continuous sequence of characters which is located to the right of the character or longest continuous sequence of characters excluded in step (xx) which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

  (xxii) excluding each character and/or each longest continuous sequence of characters selected in step (xxi) from subsequent steps of selecting a character or longest continuous sequence of characters which is the same in the reverse complementary first list of characters and the reverse complementary second list of characters;
(xxiii)

- selecting the character or longest continuous sequence of characters which is located in the continuous sequence of characters to the immediate left of each character or each longest continuous sequence of characters excluded in the previous step which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
- selecting the character or longest continuous sequence of characters which is located in the continuous sequence of characters to the immediate right of each character or each longest continuous sequence of characters excluded in the previous step which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxiv) excluding each character and/or each longest continuous sequences of characters selected in step (xxiii) from subsequent steps of selecting a character or longest continuous sequence of characters which is the same in the reverse complementary first list of characters and the reverse complementary second list of characters;
(xxv) repeating steps (xxiii) and (xxiv) until no character or longest continuous sequence of characters which is the same in the reverse complementary first list of characters and the reverse complementary second list of characters is selected;
(xxvi) adding up

- the number of characters in the reverse complementary first list of characters which were excluded in any of the steps (xix) to (xxv); and
- the number of characters in the reverse complementary second list of characters which were excluded in any of the steps (xix) to (xxv) to obtain the total number of characters, $C_c$, in the reverse complementary first and second lists of characters which are the same as in the reverse complementary second and first lists of characters, respectively;

(xxvii) adding up

- $C_c$; and
- the number of characters in the reverse complementary first list of characters which are located between the characters and/or longest continuous sequences of characters that were excluded from the reverse complementary first list of characters, and which were not excluded in any of the steps (xix) to (xxv) of step (c); and
- the number of characters in the reverse complementary second list of characters which are located between the characters and/or longest continuous sequences of characters that were excluded from the reverse complementary second list of characters, and which were not excluded in any of the steps (xix) to (xxv) of step (c),

to obtain the total number of characters, $C_t$, in the reverse complementary first and second lists of characters; and
(xxviii) calculating DS according to the following formula:

$$DS_{rcs} = C_c / C_t$$

or by:

(xxix) selecting the character or longest continuous sequence of characters which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected;
(xxx) excluding the character or longest continuous sequence of characters selected in step (xxix) from subsequent steps of selecting a character or longest continuous sequence of characters which is the same in the reverse complementary first list of characters and the reverse complementary second list of characters;
(xxxi)

- selecting the character or longest continuous sequence of characters which is located to the left of the character or longest continuous sequence of characters excluded in step (xxx) which are the same in the reverse complementary first list of characters and the reverse complementary second list of characters, wherein when two or more characters or two or more longest continuous sequences of the same length are selected, only the character or longest continuous sequence of characters which is most to the right in the reverse complementary lists of characters is selected; and
- selecting the character or longest continuous sequence of characters which is located to the right of the character or longest continuous sequence of characters excluded in step (xxx) which are the same in the reverse complementary first list of characters and the reverse complementary second list of characters, wherein when two or more characters or two or more longest continuous sequences of the same length are selected, only the character or longest continuous sequence of characters which is most to the left in the reverse complementary lists of characters is selected;

(xxxii) excluding each character and/or each longest continuous sequence of characters selected in step (xxxi) from subsequent steps of selecting a character or longest continuous sequence of characters which is the same in the reverse complementary first list of characters and the reverse complementary second list of characters;
(xxxiii) repeating steps (xxxi) and (xxxii) until no character or longest continuous sequence of characters which is the same in the reverse complementary first list of characters and the reverse complementary second list of characters is selected;
(xxxiv) adding up

- the number of characters in the reverse complementary first list of characters which were excluded in any of the steps (xxix) to (xxxiii); and
- the number of characters in the reverse complementary second list of characters which were excluded in any of the steps (xxix) to (xxxiii) to obtain the total number of characters, $C_c$, in the reverse complementary first and second lists of characters which are the same as in the reverse complementary second and first lists of characters, respectively;

(xxxv) adding up

- $C_c$; and
- the number of characters in the reverse complementary first list of characters which are located between the characters and/or longest continuous sequences of characters that were excluded from the reverse complementary first list of characters, and which were not excluded in any of the steps (xxix) to (xxxiii) of step (c); and
- the number of characters in the reverse complementary second list of characters which are located between the characters and/or longest continuous sequences of characters that were excluded from the reverse complementary second list of characters, and which were not excluded in any of the steps (xxix) to (xxxiii) of step (c),

to obtain the total number of characters, $C_t$, in the reverse complementary first and second lists of characters; and
(xxxvi) calculating DS according to the following formula:

$$DS_{rcs} = C_c / C_t$$

wherein when DS is determined for each first list of characters obtained in step (a) using sub-steps (i) to (x), $DS_{rcs}$ is determined for each corresponding reverse complementary first list of characters using sub-steps (xix) to (xxviii), and when DS is determined for each first list of characters obtained in step (a) using sub-steps (xi) to (xviii), $DS_{rcs}$ is determined for each corresponding reverse complementary first list of characters using sub-steps (xxix) to (xxxvi); and selecting, for each first list of characters obtained in step (a) and its corresponding reverse complementary first list of characters, the DS or $DS_{rcs}$ of highest value, $DS_{HV}$;

(e) adding up the number of first lists of characters which have a $DS_{HV}$ that is greater than a threshold value, T, to obtain the total number of first lists of characters, $L_c$, which are the same as a second list of characters;
(f) adding up

- $L_c$; and
- the number of first lists of characters which do not have a $DS_{HV}$ that is greater than T,

to obtain the total number of first lists of characters, $L_t$; and
(g) calculating the level of minimal residual disease (MRD) according to the following formula:

$$MRD = L_c \times (D / k) / L_t^2;$$

wherein in sub-steps (iii) and (xiii) of step (c), and sub-steps (xxi) and (xxxi) of step (d) of the present invention, selection is preferably repeated simultaneously for the continuous sequence of characters which is located to the left of the character or longest continuous sequence of characters excluded in sub-steps (ii) and (xii) of step (c), and sub-steps (xx) and (xxx) of step (d), respectively, and for the continuous sequence of characters which is located to the right of the character or longest continuous sequence of characters excluded in sub-steps (ii) and (xii), and sub-steps (xx) and (xxx) of step (d), respectively; and
wherein in sub-step (v) of step (c) and sub-step (xxiii) of step (d) of the present invention, selection is preferably repeated simultaneously for the continuous sequence of characters to the immediate left of each character or each longest continuous sequence of characters excluded in the previous step, and for the continuous sequence of characters to the immediate right of each character or each longest continuous sequence of characters excluded in the previous step.

The present disclosure also includes a kit for quantifying the level of MRD in a subject who has been treated for said disease. In accordance with the foregoing, said kit comprises:

- means for amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- means for sequencing said at least one nucleotide sequence to obtain at least one first list of characters reading from left to right.

Analogously, said kit comprises:

- means for amplifying by polymerase chain reaction using the same primers as in step (a), at least one nucleotide sequence in a biological sample obtained from said subject prior to treatment for said disease; and
- means for sequencing said at least one nucleotide sequence to obtain at least one second list of characters reading from left to right.

The means for amplifying a nucleotide sequence by polymerase chain reaction comprises a PCR instrument which operates according to any of the aforementioned techniques (preferably an emulsion PCR instrument) and primers according to the aforementioned disclosure. Likewise, the means for sequencing at least one nucleotide sequence to obtain at least one first list of characters reading from left to right comprise a nucleotide sequencing instrument which operates according to any of the aforementioned techniques (preferably a massively parallel sequencing instrument). Each of the means for amplifying and sequencing said nucleotide sequence in (a) and (b) may be comprised in different instruments. Alternatively, these means may be comprised within the same instrument.
In addition, the kit of the present disclosure comprises means (c) for determining, for each first list of characters obtained in step (a), the degree of similarity with each second list of characters obtained in step (b), wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and

(xviii) calculating DS according to the following formula:

$$DS = C_c / C_t.$$

Preferably said means (c) determines DS, $C_c$ and $C_t$ by providing instructions for each of the steps (i) to (x) and (xi) to (xviii). Furthermore preferably, said means (c) carries out each of the steps (i) to (x) and (xi) to (xviii) as previously disclosed herein. In one embodiment, said means is preferably at least one computer, at least one circuit, at least one integrated circuit, at least one chip or at least one microchip.

In addition, the kit of the present disclosure comprises means (d) for selecting, for each first list of characters obtained in step (a), the DS of highest value, $DS_{HV}$, according to the foregoing. The kit of the present disclosure also comprises means (e) for adding up the number of first lists of characters which have a $DS_{HV}$ that is greater than a threshold value, T, to obtain the total number of first lists of characters, $L_c$, which are the same as a second list of characters. Moreover, the kit of the present disclosure comprises means (f) for adding up

- $L_c$; and
- the number of first lists of characters which do not have a $DS_{HV}$ that is greater than T,

to obtain the total number of first lists of characters, $L_t$. Furthermore, the kit of the present invention comprises means (g) for calculating the level of minimal residual disease (MRD) according to the following formula:

$$MRD = L_c \text{ x } (D / k) / L_t^2.$$

As for means (c), said means (d) to (g) preferably provide instructions for each of the steps disclosed therein. Thus, preferably the kit of the present invention additionally comprises instructions for calculating the level of MRD. The level of MRD is calculated according to the aforementioned disclosure of steps (c) to (g). Said instructions are preferably carried out by a human operator or at least one computer, at least one circuit, at least one integrated circuit, at least one chip or at least one microchip. In a preferred embodiment, said instructions are carried out by means (c) following input of character lists into said means. In a further preferred embodiment of the method of the invention, steps (a) to (g) are performed by the same means, wherein said means comprises at least one computer, at least one circuit, at least one integrated circuit, at least one chip or at least one microchip. Thus, in a further preferred embodiment of the kit of the invention, means (a), (b) and (c) are part of the same means, which optionally comprises instructions (d), (e), (f) and (g).

The present invention also relates to use of the method of the invention or the kit of the invention, according to the aforementioned disclosure, in quantifying the level of minimal residual disease (MRD) in a subject who has been treated for said disease.

## Examples

The following examples illustrate the invention and should not be considered as limiting, but rather illustrative of the invention.

*Materials and Methods*

i) Samples

16 diagnostic bone marrow samples and 24 follow-up bone marrow samples obtained from subjects with said disease (test samples) were used and the series was expanded to 120 cases included in two consecutive clinical assays of Spanish myeloma patients.

ii) DNA extraction and quantification from sample

DNA samples were obtained from bone marrow biopsies using the kit QIAamp DNA mini kit (Qiagen). The quantity and quality (purity) of DNA was determined using NanoDrop 1000 (Thermo Scientific).

iii) Calculation of concentration of DNA corresponding to a desired number of equivalent cells per sample

The concentration of DNA ([DNA], pg/μL) in the biological sample obtained from a subject treated for said disease (follow-up or test sample) was measured using Qubit® dsDNA HS Assay Kit. This value was used to determine the number of equivalent cells per microliter (N) of the test sample according to the following formula:

$$N = [DNA] / k$$

wherein N and [DNA] are as defined above, and k refers to a value of 6.49 pg of DNA per diploid cell.

The number of equivalent cells per microliter (N) of the test sample allowed calculation of the volume of sample (V, μL) which it was necessary to use in the PCR in order to reach a desired sensitivity (S) according to the following formula:

$$V = 1 / (N \times S)$$

The sensitivity was determined by serial dilutions of samples (starting from 1 μg of DNA which was used to ensure a sensitivity of $10^{-5}$ or more) with known quantification of MRD on a polyclonal background (using a pool of healthy controls). A sensitivity of $10^{-5}$ equates with that achievable from use of DNA from at least 100,000 equivalent cells. The volume of sample (V) determines the number of PCR experiments necessary to obtain sufficient DNA, since each PCR experiment performed in tubes (Eppendorf tubes) with a final volume of 100 μL allows up to 8 μL of sample to be used. For example, if the number of equivalent cells per microliter (N) is 6500 and a sensitivity (S) of $10^{-5}$ is desired, it would be necessary to use $1 / (6500 \times 10^{-5}) = 15.4$ μL of test sample in the PCR, and because up to 8 μL of sample can be used per PCR experiment, two PCR experiments would be needed.

iv) PCR of the samples

PCR was performed using Platinum® Taq DNA Polymerase High Fidelity (Life Technologies) and the primers used were those described in BIOMED-2 protocol for IgH (CDR1/CDR2/CDR3 and DH) and IgK (KVJ-JK, KVJ-KDEL, INTR-KDEL), because these fragments cover more than 90% of cases (Van Dongen Leukemia 2003). The primers used to amplify said fragments of the IgH gene are shown in **Table 1,** while the primers used to amplify said fragments of the IgK gene are shown in **Table 2.**

**Table 1:** Primers for amplification of fragments of IgH

| Family primers | Tube | Sequence identifier | Primer name | Sequence | Sense |
|---|---|---|---|---|---|
| **$V_H$** | Tube A (CDR1) | SEQ ID NO:1 | $V_H$1-FR1 | GGCCTCAGTGAAGGTCTCCTGCAAG | Forward |
| | | SEQ ID NO:2 | $V_H$2-FR1 | GTCTGGTCCTACGCTGGTGAAACCC | |
| | | SEQ ID NO:3 | $V_H$3-FR1 | CTGGGGGGTCCCTGAGACTCTCCTG | |
| | | SEQ ID NO:4 | $V_H$4-FR1 | CTTCGGAGACCCTGTCCCTCACCTG | |
| | | SEQ ID NO:5 | $V_H$5-FR1 | CGGGGAGTCTCTGAAGATCTCCTGT | |
| | | SEQ ID NO:6 | $V_H$6-FR1 | TCGCAGACCCTCTCACTCACCTGTG | |
| | Tube B (CDR2) | SEQ ID NO:7 | $V_H$1-FR2 | CTGGGTGCGACAGGCCCCTGGACAA | |
| | | SEQ ID NO:8 | $V_H$2-FR2 | TGGATCCGTCAGCCCCCAGGGAAGG | |
| | | SEQ ID NO:9 | $V_H$3-FR2 | GGTCCGCCAGGCTCCAGGGAA | |
| | | SEQ ID NO: 10 | $V_H$4-FR2 | TGGATCCGCCAGCCCCCAGGGAAG G | |
| | | SEQ ID NO: 11 | $V_H$5-FR2 | GGGTGCGCCAGATGCCCGGGAAAG G | |

(continued)

| Family primers | Tube | Sequence identifier | Primer name | Sequence | Sense |
|---|---|---|---|---|---|
| | | SEQ ID NO: 12 | $V_H$6-FR2 | TGGATCAGGCAGTCCCCATCGAGAG | |
| | | SEQ ID NO: 13 | $V_H$7-FR2 | TTGGGTGCGACAGGCCCCTGGACAA | |
| | Tube C (CDR3) | SEQ ID NO: 14 | $V_H$1-FR3 | TGGAGCTGAGCAGCCTGAGATCTGA | |
| | | SEQ ID NO: 15 | $V_H$2-FR3 | CAATGACCAACATGGACCCTGTGGA | |
| | | SEQ ID NO: 16 | $V_H$3-FR3 | TCTGCAAATGAACAGCCTGAGAGCC | |
| | | SEQ ID NO: 17 | $V_H$4-FR3 | GAGCTCTGTGACCGCCGCGGACACG | |
| | | SEQ ID NO: 18 | $V_H$5-FR3 | CAGCACCGCCTACCTGCAGTGGAGC | |
| | | SEQ ID NO: 19 | $V_H$6-FR3 | GTTCTCCCTGCAGCTGAACTCTGTG | |
| | | SEQ ID NO: 20 | $V_H$7-FR3 | CAGCACGGCATATCTGCAGATCAG | |
| **$D_H$** | Tube D | SEQ ID NO: 21 | $D_H$1 | GGCGGAATGTGTGCAGGC | |
| | | SEQ ID NO: 22 | $D_H$2 | GCACTGGGCTCAGAGTCCTCT | |
| | | SEQ ID NO: 23 | $D_H$3 | GTGGCCCTGGGAATATAAAA | |
| | | SEQ ID NO: 24 | $D_H$4 | AGATCCCCAGGACGCAGCA | |
| | | SEQ ID NO: 25 | $D_H$5 | CAGGGGGACACTGTGCATGT | |
| | | SEQ ID NO: 26 | $D_H$6 | TGACCCCAGCAAGGGAAGG | |
| | Tube E | SEQ ID NO: 27 | $D_H$7 | CACAGGCCCCCTACCAGC | |
| **$J_H$** | Tubes A-E | SEQ ID NO: 28 | JH57 | CTTACCTGAGGAGACGGTGACC | Reverse |

**Table 2:** Primers for amplification of fragments of IgK

| Family primers | Tube | Sequence identifier | Primer name | Sequence | Sense |
|---|---|---|---|---|---|
| $V_K$ | Tube F, G | SEQ ID NO:29 | $V_K$1f/6 | TCAAGGTTCAGCGGCAGTGGATCTG | Forward |
| | | SEQ ID NO:30 | $V_K$2f | GGCCTCCATCTCCTGCAGGTCTAGTC | |
| | | SEQ ID NO:31 | $V_K$3f | CCCAGGCTCCTCATCTATGATGCATCC | |
| | | SEQ ID NO:32 | $V_K$4 | CAACTGCAAGTCCAGCCAGAGTGTTTT | |
| | | SEQ ID NO:33 | $V_K$5 | CCTGCAAAGCCAGCCAAGACATTGAT | |
| | | SEQ ID NO:34 | $V_K$6 | GACCGATTTCACCCTCACAATTAATCC | |
| $J_K$ | Tube F | SEQ ID NO:35 | $J_K$1-4 | CTTACGTTTGATCTCCACCTTGGTCCC | Reverse |
| | | SEQ ID NO:36 | $J_K$5 | CTTACGTTTAATCTCCAGTCGTGTCCC | |
| KDEL | Tube G, H | SEQ ID NO:37 | KDEL | CCTCAGAGGTCAGAGCAGGTTGTCCT A | |
| $J_K$- $C_K$ Intron | Tube H | SEQ ID NO:38 | INTR | CGTGGCACCGCGAGCTGTAGAC | Forward |

Amplification of the test simple was performed using the number of PCR experiments (*i.e.* PCR tubes) which were calculated as necessary based on the volume of the test sample (V, µL), the number of equivalent cells per microliter (N) of said sample, the sensitivity (S) which it was desired to reach and the final PCR tube volume. Accordingly, the amounts of each component (per PCR tube) for each PCR reaction mix for the test sample were as follows:

a) PCR CDR1/CDR2/CDR3

84 µL Platinum HIFI master mix
4 µL Primers CDR1/CDR2/CDR3 mix (Tubes A, B, C)
4 µL Primer JH57
8 µL gDNA

b) PCR KVJ

80 µL Platinum HIFI master mix
4 µL Primers KVmix (Tube F)
4 µL Primer KJ5
4 µL Primer KJ1-4
8 µL gDNA

c) PCR DH

84 µL Platinum HIFI master mix
4 µL Primers DH 1-6 (Tube D) or DH7 (Tube E)
4 µL JH57
8 µL gDNA

d) PCR KDEL

80 μL Platinum HIFI master mix
4 μL Primers KVmix (Tube G)
4 μL INTR (Tube H)
4 μL KDEL
8 μL gDNA

The diagnostic sample is amplified with the same reactions but using 1 μL of DNA (1 μL of DNA = approximately 20ng gDNA, *i.e.* [DNA] = 20 μg/mL) because it is not necessary to reach a given sensitivity in the diagnostic sample.

v) Preparation of amplicon libraries without fragmentation

The amplifed products of both samples (diagnosis and follow-up) were used to prepare respective amplicon libraries without fragmentation using Ion Plus Fragment Library kit and Agencourt Ampure XP (Thermo-Fisher). It was found possible to use half of the volumes of all reactants using the Ion Plus Fragment Library kit. The final library concentration was determined using qPCR in the GeneRead Library Quant kit (Qiagen). The libraries were generated using specific barcodes for each sample.

vi) Massive parallel sequencing

The main clone or clones were identified in the diagnostic sample *via* massive parallel sequencing of the product of the amplification of CDR1, CDR2, CD3, KVJ, DH and KDEL. Clonal samples with fragments greater than 250 bp were sequenced on the PGM platform (Ion Torrent Personal Genome Machine™ platform) using OneTouch™ Ion v2 Kit Template 400 DL, 400 Ion PGM™ Sequencing Kit v2 and Ion Chip 318™ Kit according to the manufacturer's instructions (Thermo-Fisher). Fragments less than 250 bp (mainly from samples with the rearrangements KVJ and KDEL) were sequenced using the PROTON platform: Ion Proton™I emulsion OT2 Template Kit and sequencing Ion Proton™ I Sequencing Kit (Thermo-Fisher). All reagents were purchased from Lifetech using their protocols with slight modifications: PGM platform technology sequences fragments up to 250 bp, but it is possible to sequence fragments up to 400 bp with another commercial kit of Lifetech using different chemistry.

vii) Bioinformatic analysis

After sequencing, FASTQ files of the two samples were obtained from the Torrent Browser according to the corresponding Barcode. Each FASTQ file comprises a list of characters reading from left to right which represents the nucleotide sequence of the DNA comprised in said sample, and additionally comprises the quality score corresponding to each character of said list of characters.

The quantification of each clonotypic sequence or sequences in the diagnostic sample was determined using mathematical and computer methods (IT tools), namely using the FrequencyRank.sh Bourne shell script (frequency_rank.sh) to sort sequences in descending frequency order. Once the clonal sequences which are the same in the diagnostic sample as in the follow-up sample were determined, a .dna file was generated comprising each of said clonal sequences as a list of characters reading from left to right and having a total number of characters

The number of clonal sequences (first lists of characters) identified in the diagnostic sample which were considered the same as the argument sequence (second lists of characters was counted using the SeqSearchFastq.java program with the -trim option and a match ratio (degree of similarlity) of 0.99, to give a value, $L_c$. $L_t$ was determined from the total number of first lists of characters.

As the method of the invention involves a mixture of alignment and comparison, comparison was made only between the first and the last matching position and the - trim option instructs the process to act in this way, limiting the comparison from the first and last matching positions instead of first and last positions (regardless of matching) in the sample sequence. The output, $L_c$, from the SeqSearchFastq.java program is subsequently used, together with the values for $L_t$, k and D, to calculate the MRD.

*Example 1. Quantification of MRD in multiple myeloma using massively parallel sequencing of genes of immunoglobulins*

The following presents a method for quantification of tumor clonotypic sequences within the polyclonal background rearrangements of genes of immunoglobulins (Ig) *via* massively parallel sequencing (MPS). The detection of clonal rearrangement in B and T cell neoplasms allows the evolution of these pathologies to be monitored. To quantify these rearrangements in B cells, primers disclosed in **Tables 1** and **2** for CDR3, VDJ, IgH, IgK, KVJ, KDEL, and IgL were used, because these fragments cover more than 90% of cases (Van Dongen, Leukemia 2003). The selection of these particular rearrangements is due to the design of primers which only amplify short (less than 200 bp) sequences; allowing to sequence these fragments in the PROTON platform, capable of 10 Gb.

Patients negative for VDJ, IgH, GDR3, KVJ, KDEL diagnoses may be sequenced with the rest of the BIOMED primers like IgH, VDJ, CDRI and IgL DJ. As the size of these fragments is between 300 and 400 base pairs (bp), it is necessary to use the PGM platform with reactive kit for 400 bp. The ability of PGM is near 1 Gbase, whereby ability refers to the number of bases that can be read in a PGM run.

*Results:*

Serial dilutions of clonotypic experimental samples indicated a sensitivity of $10^{-5}$ for 150,000 cells. The correlation of all samples with the data from flow cytometry achieved an $R = 0.59$ (Pearson = 0.765, $p<0.0001$) and $R = 0.51$ (Pearson = 0.716, $p<0.0001$) for follow-up samples. The average of the readings of the BIOMED primers was similar to the frequencies of the different fragments described in multiple myeloma. The reproducibility of the technique was over 90%. Of the 24 follow-ups analyzed, two were positive by massively parallel sequencing and negative by flow cytometry and one was negative by massively parallel sequencing and positive by flow cytometry. In the PGM platform, parallel analysis of 12 follow-ups was achieved in a week with a coverage of 500.000x and at an approximate price of 100 Euros/sample.

In **Figure 3,** the sensitivity achieved in serial dilutions of multiple myeloma clonal rearrangements on a polyclonal background obtained from healthy patients or from non B cell lines, is shown. The sensitivity in the characterization of gene rearrangements of immunoglobulins is determined by the amount of input DNA, or equivalent cell number. The sensitivity of this technique depends on the number of variants found in a clonal reading and shows that the method of the invention exhibits extremely high sensitivity in detection of MRD.

Figure 4 shows the main clonal rearrangement correlation in samples from patients who have been treated for multiple myeloma (follow-up samples) between MRD data measured with flow cytometry (y axis) and massive parallel sequencing according to the present invention (x axis). MRD quantification in multiple myeloma performed by flow cytometry refers to the total cell number, with equivalent equation to that of this invention. The correlation between the two techniques is high, with $R^2 = 0.51$.

*Conclusions*:

Deep-sequencing of the rearrangements of Ig genes by Ion Torrent technology is an effective technique to define and quantify the pathological clones in multiple myeloma. This technique is a methodical and economically viable alternative to flow cytometry and other methods of monitoring MRD.

Thus, quantitation with high sensitivity of the specific sequences belonging to the pathological clones that define the condition allows the monitoring of the evolution and cellular response to specific treatments, the definition of new disease foci, and the monitoring of minimal residual disease in patients with defined genetic alterations.

*Example 2. Quantification of SNV, MNV and indels*

The method described in the foregoing is applicable to the detection of any type of mutation, given some limitations, as follows. The average error based on massive sequencing platforms is 0.5% or, in other words, one erroneous reading in 200 for each position in the genome. The probability that an error happens reading the variant sought is 0.5% / 4 bases, or about 0.1 %. This theoretical limitation has been verified experimentally for point mutations (SNV: DNM3A and IDH2) in cases of AML (acute myeloid leukemia, **Figure 5),** wherein this error is 0.1% for each position. In those mutations that include more than two positions for reading, such as a multiple mutation (MNV) or an indel, the error will be (0.1 x n)%, where n is the number of clonal variants present in the reading.

*Example 3. Quantification of long insertions and translocations*

In this case the sensitivity limit is not reached due to the fact that there is no background against which to compare readings, because primers amplify only those DNA fragments that have a translocation or inversion. To alleviate this problem, a control DNA was used, this being one of the genes involved in the translocation, in its wild-type form. Thus the ratio of clonotypic sequences / total sequences takes into account the number of readings of both.

## Claims

**1.** A method for quantifying the level of minimal residual disease (MRD) in a subject who has been treated for said disease, which comprises:

(a)

- amplifying by polymerase chain reaction using primers, at least one nucleotide sequence comprised in an amount, D, of genomic DNA of a biological sample obtained from said subject after treatment for said disease, wherein the genomic DNA has an average weight, k, per diploid cell of said biological sample; and
- sequencing said at least one nucleotide sequence to obtain at least one first list of characters reading from left to right;

(b)

- amplifying by polymerase chain reaction using the same primers as in step (a), at least one nucleotide sequence in a biological sample obtained from said subject prior to treatment for said disease; and
- sequencing said at least one nucleotide sequence to obtain at least one second list of characters reading from left to right;

(c) determining, for each first list of characters obtained in step (a), the degree of similarity with each second list of characters obtained in step (b), wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and
- the number of characters in the second list of characters which were excluded in any of the steps (i) to (vii)

to obtain the total number of characters, $C_c$, in the first and second lists of characters which are the same as in the second and first lists of characters, respectively;
(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and
(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(xii) excluding the character or longest continuous sequence of characters selected in step (xi) from subsequent steps of selecting a character or longest continuous sequence of characters which is the same in the first list of characters and the second list of characters;
(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;
(xv) repeating steps (xiii) and (xiv) until no character or longest continuous sequence of characters which is the same in the first list of characters and the second list of characters is selected;
(xvi) adding up

- the number of characters in the first list of characters which were excluded in any of the steps (xi) to (xv); and
- the number of characters in the second list of characters which were excluded in any of the steps (xi) to (xv)

to obtain the total number of characters, $C_c$, in the first and second lists of characters which are the same as in the second and first lists of characters, respectively;
(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and
(xviii) calculating DS according to the following formula:

$$DS = C_c / C_t$$

(d) selecting, for each first list of characters obtained in step (a), the DS of highest value, $DS_{HV}$;

(e) adding up the number of first lists of characters which have a $DS_{HV}$ that is greater than a threshold value, T, to obtain the total number of first lists of characters, $L_c$, which are the same as a second list of characters;
(f) adding up

- $L_c$; and
- the number of first lists of characters which do not have a $DS_{HV}$ that is greater than T,

to obtain the total number of first lists of characters, $L_t$; and
(g) calculating the level of minimal residual disease (MRD) according to the following formula:

$$MRD = L_c \times (D / k) / L_t^2.$$

**2.** The method according to claim 1, wherein

- each character in each of the first and second lists of characters respectively obtained in steps (a) and (b), comprises a letter associated with a number or symbol, wherein said number or symbol represents quality (*Q*), wherein quality (*Q*) is an integer mapping of the probability that the letter which represents a nucleotide that is identified at the corresponding position in the nucleotide sequence is incorrect, and wherein said letter represents the nucleotide that is identified at the corresponding position in the nucleotide sequence having the highest quality (*Q*); and
- a character in the first list of characters is determined in step (c) as the same as a character in the second list of characters, when the letter having the highest quality is the same in the first and second lists of characters.

**3.** The method according to claim 2, wherein a character in the first list of characters is determined in step (c) as the same as a character in the second list of characters, when the letter having the highest quality (*Q*) is the same in the first and second lists of characters, and the quality of the letter in the first list of characters is within 1 percent of the quality of the letter in the second list of characters.

**4.** The method according to any of the previous claims 1 to 3, wherein the sequencing is massively parallel sequencing.

**5.** The method according to any of the previous claims 1 to 4, wherein an MRD of 1 is 100% indicative of disease in said subject and an MRD of 0 is 0% indicative of disease in said subject.

**6.** The method according to any of the previous claims 1 to 5, wherein the disease is selected from cancer or leukaemia.

**7.** The method according to claim 6, wherein the disease is selected from acute lymphoblastic leukaemia, acute myeloid leukaemia, chronic lymphocytic leukaemia, chronic myelogenous leukemia, follicular lymphoma, mantle cell lymphoma, multiple myeloma, breast cancer or neuroblastoma.

**8.** A kit for quantifying the level of minimal residual disease (MRD) in a subject who has been treated for said disease which comprises:

(a)

- means for amplifying in a PCR instrument, by polymerase chain reaction, at least one nucleotide sequence comprised in an amount, D, of genomic DNA of a biological sample obtained from said subject after treatment for said disease, wherein the genomic DNA has an average weight, k, per diploid cell of said biological sample, wherein said means comprise locus-specific forward and locus-specific reverse, primers, to identify one specific variant of a nucleotide sequence present in the biological sample, wherein said one variant or the absence of said one variant is indicative of disease; and
- means for sequencing said at least one nucleotide sequence to obtain at least one first list of characters reading from left to right, wherein the sequencing comprises a massively parallel sequencing platform;

(b)

- means for amplifying, by polymerase chain reaction, in a PCR instrument, using the same locus-specific primers as in step (a), at least one nucleotide sequence in a biological sample obtained from said subject prior to treatment for said disease; and
- means for sequencing said at least one nucleotide sequence to obtain at least one second list of characters reading from left to right, wherein the sequencing comprises a massively parallel sequencing platform;

(c) means for determining, for each first list of characters obtained in step (a), the degree of similarity with each second list of characters obtained in step (b), wherein said means comprise preferably at least one computer, at least one circuit, at least one integrated circuit, at least one chip or at least one microchip, and wherein a degree of similarity, DS, of a first list of characters obtained in step (a) with a second list of characters obtained in step (b) is determined either by:

(i) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;
(ii) excluding the character or longest continuous sequence of characters selected in step (i) from subsequent steps of selecting a character or longest continuous sequence of characters which is the same in the first list of characters and the second list of characters;
(iii)

- selecting the character or longest continuous sequence of characters which is located to the left of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (ii) which are the same in the first list of characters and the second list of characters, wherein when two or more characters or two or more longest continuous sequences of the same length are selected, only the character or longest continuous sequence of characters which is most to the left in the lists of characters is selected;

(iv) excluding each character and/or each longest continuous sequence of characters selected in step (iii) from subsequent steps of selecting a character or longest continuous sequence of characters which is the same in the first list of characters and the second list of characters;
(v)

- selecting the character or longest continuous sequence of characters which is located in the continuous sequence of characters to the immediate left of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located in the continuous sequence of characters to the immediate right of each character or each longest continuous sequence of characters excluded in the previous step which are the same in the first list of characters and the second list of characters, wherein when two or more characters or two or more longest continuous sequences of the same length are selected, only the character or longest continuous sequence of characters which is most to the left in the lists of characters is selected;

(vi) excluding each character and/or each longest continuous sequences of characters selected in step (v) from subsequent steps of selecting a character or longest continuous sequence of characters which is the same in the first list of characters and the second list of characters;
(vii) repeating steps (v) and (vi) until no character or longest continuous sequence of characters which is the same in the first list of characters and the second list of characters is selected;
(viii) adding up

- the number of characters in the first list of characters which were excluded in any of the steps (i) to (vii); and

- the number of characters in the second list of characters which were excluded in any of the steps (i) to (vii)

to obtain the total number of characters, $C_c$, in the first and second lists of characters which are the same as in the second and first lists of characters, respectively;

(ix) adding up

- $C_c$; and
- the number of characters in the first list of characters which are located between the characters and/or longest continuous sequences of characters that were excluded from the first list of characters, and which were not excluded in any of the steps (i) to (vii) of step (c); and
- the number of characters in the second list of characters which are located between the characters and/or longest continuous sequences of characters that were excluded from the second list of characters, and which were not excluded in any of the steps (i) to (vii) of step (c),

to obtain the total number of characters, $C_t$, in the first and second lists of characters; and

(x) calculating DS according to the following formula:

$$DS = C_c / C_t$$

or by:

(xi) selecting the character or longest continuous sequence of characters which are the same in the first list of characters and the second list of characters, wherein when two or more characters or two or more longest continuous sequences of the same length are selected, only the character or longest continuous sequence of characters which is most to the right in the lists of characters is selected;

(xii) excluding the character or longest continuous sequence of characters selected in step (xi) from subsequent steps of selecting a character or longest continuous sequence of characters which is the same in the first list of characters and the second list of characters;

(xiii)

- selecting the character or longest continuous sequence of characters which is located to the left of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the right in the lists of characters is selected; and
- selecting the character or longest continuous sequence of characters which is located to the right of the character or longest continuous sequence of characters excluded in step (xii) which are the same in the first list of characters and the second list of characters, wherein when two or more characters or two or more longest continuous sequences of the same length are selected, only the character or longest continuous sequence of characters which is most to the left in the lists of characters is selected;

(xiv) excluding each character and/or each longest continuous sequence of characters selected in step (xiii) from subsequent steps of selecting a character or longest continuous sequence of characters which is the same in the first list of characters and the second list of characters;

(xv) repeating steps (xiii) and (xiv) until no character or longest continuous sequence of characters which is the same in the first list of characters and the second list of characters is selected;

(xvi) adding up

- the number of characters in the first list of characters which were excluded in any of the steps (xi) to (xv); and
- the number of characters in the second list of characters which were excluded in any of the steps (xi) to (xv) to obtain the total number of characters, $C_c$, in the first and second lists of characters which are the same as in the second and first lists of characters, respectively;

(xvii) adding up

- $C_c$; and
- the number of characters in the first list of characters which are located between the characters and/or longest continuous sequences of characters that were excluded from the first list of characters, and which were not excluded in any of the steps (xi) to (xv) of step (c); and
- the number of characters in the second list of characters which are located between the characters and/or longest continuous sequences of characters that were excluded from the second list of characters, and which were not excluded in any of the steps (xi) to (xv) of step (c),

to obtain the total number of characters, $C_t$, in the first and second lists of characters; and
(xviii) calculating DS according to the following formula:

$$DS = C_c / C_t$$

(d) means or instructions for selecting, for each first list of characters obtained in step (a), the DS of highest value, $DS_{HV}$;
(e) means or instructions for adding up the number of first lists of characters which have a $DS_{HV}$ that is greater than a threshold value, T, to obtain the total number of first lists of characters, $L_c$, which are the same as a second list of characters;
(f) means or instructions for adding up

- $L_c$; and
- the number of first lists of characters which do not have a $DS_{HV}$ that is greater than T,

to obtain the total number of first lists of characters, $L_t$; and
(g) means and instructions for calculating the level of minimal residual disease (MRD) according to the following formula:

$$MRD = L_c \text{ x } (D / k) / L_t^2;$$

and wherein said means of steps (a) to (g) are able to calculate MRD, or they provide instructions to calculate MRD, without the need to access external databases; and wherein said means comprise: at least one computer, or at least one circuit, or at least one integrated circuit, or at least one chip, or at least one microchip; and wherein said instructions can be carried out: by a human operator, or at least one computer, or at least one circuit, or at least one integrated circuit, or at least one chip, or at least one microchip.

**9.** The kit according to claim 8, wherein

- each character in each of the first and second lists of characters respectively obtained in steps (a) and (b), comprises a letter associated with a number or symbol, wherein said number or symbol represents quality (*Q*), wherein quality (*Q*) is an integer mapping of the probability that the letter which represents a nucleotide that is identified at the corresponding position in the nucleotide sequence is incorrect, and wherein said letter represents the nucleotide that is identified at the corresponding position in the nucleotide sequence having the highest quality (*Q*); and
- a character in the first list of characters is determined in (c) as the same as a character in the second list of characters, when the letter having the highest quality is the same in the first and second lists of characters.

**10.** The kit according to claim 9, wherein a character in the first list of characters is determined as the same as a character in the second list of characters, when the letter having the highest quality (*Q*) is the same in the first and second lists of characters, and the quality of the letter in the first list of characters is within 1 percent of the quality of the letter in the second list of characters.

**11.** The kit according to any of the previous claims 8 to 10, wherein an MRD of 1 is 100% indicative of disease in said subject and an MRD of 0 is 0% indicative of disease in said subject.

**12.** The kit according to any of the previous claims 8 to 11, wherein the disease is selected from cancer or leukaemia.

**13.** The kit according to claim 12, wherein the disease is selected from acute lymphoblastic leukaemia, acute myeloid leukaemia, chronic lymphocytic leukaemia, chronic myelogenous leukemia, follicular lymphoma, mantle cell lymphoma, multiple myeloma, breast cancer or neuroblastoma.

**14.** The kit according to claim 8 wherein the means for sequencing used in steps (a) and/or (b) are selected from: multiplex and/or high-throughput nucleotide sequencing; massively parallel signature sequencing (MPSS) and massively parallel sequencing using emulsion-PCR.

**15.** Use of the method according to any of claims 1 to 7 or the kit according to any of claims 8 to 14 in quantifying the level of minimal residual disease (MRD) in a subject who has been treated for said disease.

**Patentansprüche**

**1.** Verfahren zum Quantifizieren des Grads der minimalen Resterkrankung ("minimal residual disease", MRD) bei einem Individuum, das wegen dieser Erkrankung behandelt wurde, umfassend:

(a)

- Amplifizieren zumindest einer Nukleotidsequenz, die in einer Menge D von genomischer DNA einer dem Individuum nach der Behandlung wegen der Erkrankung entnommenen biologischen Probe enthalten ist, durch Polymerasekettenreaktion unter Verwendung von Primern, wobei die genomische DNA ein durchschnittliches Gewicht k pro diploider Zelle der biologischen Probe besitzt; und
- Sequenzieren der zumindest einen Nukleotidsequenz, um zumindest eine erste Liste von Zeichen zum Lesen von links nach rechts zu erhalten;

(b)

- Amplifizieren zumindest einer Nukleotidsequenz in einer dem Individuum vor der Behandlung wegen der Erkrankung entnommenen biologischen Probe durch Polymerasekettenreaktion unter Verwendung derselben Primer wie in Schritt (a); und
- Sequenzieren der zumindest einen Nukleotidsequenz, um zumindest eine zweite Liste von Zeichen zum Lesen von links nach rechts zu erhalten;

(c) Bestimmen des Ähnlichkeitsgrads mit jeder in Schritt (b) erhaltenen zweiten Liste von Zeichen für jede in Schritt (a) erhaltene erste Liste von Zeichen, wobei ein Ähnlichkeitsgrad ("degree of similarity", DS) einer in Schritt (a) erhaltenen ersten Liste von Zeichen mit einer in Schritt (b) erhaltenen zweiten Liste von Zeichen auf einem der folgenden Wege ermittelt wird:

(i) Auswählen des Zeichens oder der längsten zusammenhängenden Abfolge von Zeichen, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet;
(ii) Ausschließen des Zeichens oder der längsten zusammenhängenden Abfolge von Zeichen, das bzw. die in Schritt (i) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(iii)

- Auswählen desjenigen Zeichens oder derjenigen längsten zusammenhängenden Abfolge von Zeichen, das bzw. die sich links von dem Zeichen bzw. der längsten zusammenhängenden Abfolge von Zeichen befinden, das bzw. die in Schritt (ii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet; und

- Auswählen desjenigen Zeichens oder derjenigen längsten zusammenhängenden Abfolge von Zeichen, das bzw. die sich rechts von dem Zeichen bzw. der längsten zusammenhängenden Abfolge von Zeichen befinden, das bzw. die in Schritt (ii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten links befindet;

(iv) Ausschließen jedes Zeichens und/oder jeder längsten zusammenhängenden Abfolge von Zeichen, das bzw. die im Schritt (iii) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;

(v)

- Auswählen desjenigen Zeichens oder derjenigen längsten zusammenhängenden Abfolge von Zeichen, das bzw. die sich in der zusammenhängenden Abfolge von Zeichen unmittelbar links von dem jeweiligen Zeichen bzw. der jeweiligen längsten zusammenhängenden Abfolge von Zeichen befinden, das bzw. die in vorstehendem Schritt ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet; und
- Auswählen desjenigen Zeichens oder derjenigen längsten zusammenhängenden Abfolge von Zeichen, das bzw. die sich in der zusammenhängenden Abfolge von Zeichen unmittelbar rechts von dem jeweiligen Zeichen bzw. der jeweiligen längsten zusammenhängenden Abfolge von Zeichen befinden, das bzw. die in vorstehendem Schritt ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten links befindet;

(vi) Ausschließen jedes Zeichens und/oder jeder längsten zusammenhängenden Abfolge von Zeichen, das bzw. die in Schritt (v) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;

(vii) Wiederholen der Schritte (v) und (vi), bis kein Zeichen oder keine längste zusammenhängende Abfolge von Zeichen mehr ausgewählt wird, das bzw. die in der ersten Liste von Zeichen und in der zweiten Liste von Zeichen gleich ist;

(viii) Addieren

- der Anzahl von Zeichen in der ersten Liste von Zeichen, die in jedem der Schritte (i) bis (vii) ausgeschlossen wurden; und
- der Anzahl von Zeichen in der zweiten Liste von Zeichen, die in jedem der Schritte (i) bis (vii) ausgeschlossen wurden,

um die Gesamtanzahl der Zeichen, Cc, in der ersten und der zweiten Liste von Zeichen zu erhalten, die gleich sind wie in der zweiten bzw. in der ersten Liste von Zeichen;

(ix) Addieren von

- $C_c$; und
- der Anzahl der Zeichen in der ersten Liste von Zeichen, die sich zwischen den Zeichen und/oder längsten zusammenhängenden Abfolgen von Zeichen befinden, die aus der ersten Liste von Zeichen ausgeschlossen wurden und die in keinem der Schritte (i) bis (vii) des Schrittes (c) ausgeschlossen wurden; und
- der Anzahl der Zeichen in der zweiten Liste von Zeichen, die sich zwischen den Zeichen und/oder längsten zusammenhängenden Abfolgen von Zeichen befinden, die aus der zweiten Liste von Zeichen ausgeschlossen wurden und die in keinem der Schritte (i) bis (vii) des Schrittes (c) ausgeschlossen wurden,

um die Gesamtanzahl von Zeichen, Ct, in der ersten und der zweiten Liste von Zeichen zu erhalten; und
(x) Berechnen des DS gemäß der nachstehenden Formel:

$$DS = C_c / C_t$$

oder auf folgendem Wege:

(xi) Auswählen des Zeichens oder der längsten zusammenhängenden Abfolge von Zeichen, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet;
(xii) Ausschließen des Zeichens oder der längsten zusammenhängenden Abfolge von Zeichen, das bzw. die in Schritt (xi) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(xiii)

- Auswählen desjenigen Zeichens oder derjenigen längsten zusammenhängenden Abfolge von Zeichen, das bzw. die sich links von dem Zeichen bzw. der längsten zusammenhängenden Abfolge von Zeichen befinden, das bzw. die in Schritt (xii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet; und
- Auswählen desjenigen Zeichens oder derjenigen längsten zusammenhängenden Abfolge von Zeichen, das bzw. die sich rechts von dem Zeichen bzw. der längsten zusammenhängenden Abfolge von Zeichen befinden, das bzw. die in Schritt (xii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten links befindet;

(xiv) Ausschließen jedes Zeichens und/oder jeder längsten zusammenhängenden Abfolge von Zeichen, das bzw. die in Schritt (xiii) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(xv) Wiederholen der Schritte (xiii) und (xiv), bis kein Zeichen oder keine längste zusammenhängende Abfolge von Zeichen mehr ausgewählt wird, das bzw. die in der ersten Liste von Zeichen und in der zweiten Liste von Zeichen gleich ist;
(xvi) Addieren

- der Anzahl von Zeichen in der ersten Liste von Zeichen, die in jedem der Schritte (xi) bis (xv) ausgeschlossen wurden; und
- der Anzahl von Zeichen in der zweiten Liste von Zeichen, die in jedem der Schritte (xi) bis (xv) ausgeschlossen wurden,

um die Gesamtanzahl der Zeichen, Cc, in der ersten und der zweiten Liste von Zeichen zu erhalten, die gleich sind wie in der zweiten bzw. in der ersten Liste von Zeichen;
(xvii) Addieren von

- $C_c$; und
- der Anzahl der Zeichen in der ersten Liste von Zeichen, die sich zwischen den Zeichen und/oder längsten zusammenhängenden Abfolgen von Zeichen befinden, die aus der ersten Liste von Zeichen ausgeschlossen wurden und die in keinem der Schritte (xi) bis (xv) des Schrittes (c) ausgeschlossen wurden; und
- der Anzahl der Zeichen in der zweiten Liste von Zeichen, die sich zwischen den Zeichen und/oder längsten zusammenhängenden Abfolgen von Zeichen befinden, die aus der zweiten Liste von Zeichen ausgeschlossen wurden und die in keinem der Schritte (xi) bis (xv) des Schrittes (c) ausgeschlossen wurden,

um die Gesamtanzahl von Zeichen, Ct, in der ersten und der zweiten Liste von Zeichen zu erhalten; und
(xviii) Berechnen des DS gemäß der nachstehenden Formel:

$$DS = C_c / C_t$$

(d) Auswählen des DS mit dem höchsten Wert, $DS_{HV}$, für jede in Schritt (a) erhaltene erste Liste von Zeichen;
(e) Addieren der Anzahl der ersten Listen von Zeichen mit einem $DS_{HV}$, der größer ist als ein Grenzwert T, um die Gesamtanzahl der ersten Listen von Zeichen, $L_c$, zu erhalten, die gleich sind wie eine zweite Liste von Zeichen;,
(f) Addieren von

- $L_c$; und
- der Anzahl der ersten Listen von Zeichen ohne $DS_{HV}$ der größer ist als T,

um die Gesamtanzahl von ersten Listen von Zeichen, $L_t$, zu erhalten; und
(g) Berechnen des Grads der minimalen Resterkrankung (MRD) gemäß der nachstehenden Formel:

$$MRD = L_c \times (D / k) / L_t^2.$$

**2.** Verfahren nach Anspruch 1, wobei:

- jedes Zeichen in jeder der ersten und der zweiten Liste von Zeichen, das in Schritt (a) bzw. (b) erhalten wurde, einen Buchstaben umfasst, der einer Zahl oder einem Symbol zugeordnet ist, wobei die Zahl oder das Symbol für Qualität (*Q*) steht, wobei die Qualität (*Q*) eine ganzzahlige Funktion der Wahrscheinlichkeit ist, dass der Buchstabe, der für ein Nukleotid steht, das an der entsprechenden Position in der Nukleotidsequenz identifiziert wurde, falsch ist, und wobei der Buchstabe für das Nukleotid steht, das an der entsprechenden Position in der Nukleotidsequenz mit der höchsten Qualität (*Q*) identifiziert wurde; und
- ein Zeichen in der ersten Liste von Zeichen in Schritt (c) als gleich wie ein Zeichen in der zweiten Liste von Zeichen ermittelt wird, wenn der Buchstabe mit der höchsten Qualität in der ersten und der zweiten Liste von Zeichen gleich ist.

**3.** Verfahren nach Anspruch 2, wobei ein Zeichen in der ersten Liste von Zeichen in Schritt (c) als gleich wie ein Zeichen in der zweiten Liste von Zeichen ermittelt wird, wenn der Buchstabe mit der höchsten Qualität (*Q*) in der ersten und der zweiten Liste von Zeichen gleich ist und die Qualität des Buchstabens in der ersten Liste von Zeichen innerhalb von 1 Prozent der Qualität des Buchstabens in der zweiten Liste von Zeichen liegt.

**4.** Verfahren nach einem der Ansprüche 1 bis 3, wobei die Sequenzierung massiv-parallele Sequenzierung ist.

**5.** Verfahren nach einem der Ansprüche 1 bis 4, wobei eine MRD von 1 zu 100 % auf eine Erkrankung bei dem Individuum hindeutet und eine MRD von 0 zu 0 % auf eine Erkrankung bei dem Individuum hindeutet.

**6.** Verfahren nach einem der Ansprüche 1 bis 5, wobei die Erkrankung aus Krebs oder Leukämie ausgewählt ist.

**7.** Verfahren nach Anspruch 6, wobei die Erkrankung aus akuter lymphoblastischer Leukämie, akuter myeloischer Leukämie, chronischer lymphatischer Leukämie, chronischer myeloischer Leukämie, follikulärem Lymphom, Mantelzelllymphom, multiplem Myelom, Brustkrebs oder Neuroblastom ausgewählt ist.

**8.** Set zum Quantifizieren des Grads der minimalen Resterkrankung ("minimal residual disease", MRD) bei einem Individuum, das wegen dieser Erkrankung behandelt wurde, umfassend:

(a)

- Mittel zum Amplifizieren zumindest einer Nukleotidsequenz, die in einer Menge D von genomischer DNA einer dem Individuum nach der Behandlung wegen der Erkrankung entnommenen biologischen Probe enthalten ist, durch Polymerasekettenreaktion in einem PCR-Instrument, wobei die genomische DNA ein durchschnittliches Gewicht k pro diploider Zelle der biologischen Probe besitzt, wobei die Mittel locusspezifische Vorwärts- und locusspezifische Rückwärtsprimer umfassen, um eine spezifische Variante einer in der biologischen Probe vorhandenen Nukleotidsequenz zu identifizieren, wobei die eine Variante oder das Fehlen der einen Variante auf die Erkrankung hindeutet; und
- Mittel zum Sequenzieren der zumindest einen Nukleotidsequenz, um zumindest eine erste Liste von Zeichen zum Lesen von links nach rechts zu erhalten, wobei die Sequenzierung eine Plattform für die massiv-parallele Sequenzierung umfasst;

(b)

- Mittel zum Amplifizieren zumindest einer Nukleotidsequenz in einer dem Individuum vor der Behandlung wegen der Erkrankung entnommenen biologischen Probe durch Polymerasekettenreaktion in einem PCR-Instrument unter Verwendung derselben locusspezifischen Primer wie in Schritt (a); und
- Mittel zum Sequenzieren der zumindest einen Nukleotidsequenz, um zumindest eine zweite Liste von Zeichen zum Lesen von links nach rechts zu erhalten, wobei die Sequenzierung eine Plattform für die massiv-parallele Sequenzierung umfasst;

(c) Mittel zum Bestimmen des Ähnlichkeitsgrads mit jeder in Schritt (b) erhaltenen zweiten Liste von Zeichen für jede in Schritt (a) erhaltene erste Liste von Zeichen, wobei die Mittel vorzugsweise zumindest einen Rechner, zumindest einen Schaltkreis, zumindest einen integrierten Schaltkreis, zumindest einen Chip oder zumindest einen Mikrochip umfassen und wobei ein Ähnlichkeitsgrad ("degree of similarity", DS) einer in Schritt (a) erhaltenen ersten Liste von Zeichen mit einer in Schritt (b) erhaltenen zweiten Liste von Zeichen auf einem der folgenden Wege ermittelt wird:

(i) Auswählen des Zeichens oder der längsten zusammenhängenden Abfolge von Zeichen, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet;
(ii) Ausschließen des Zeichens oder der längsten zusammenhängenden Abfolge von Zeichen, das bzw. die in Schritt (i) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(iii)

- Auswählen desjenigen Zeichens oder derjenigen längsten zusammenhängenden Abfolge von Zeichen, das bzw. die sich links von dem Zeichen bzw. der längsten zusammenhängenden Abfolge von Zeichen befinden, das bzw. die in Schritt (ii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet; und
- Auswählen desjenigen Zeichens oder derjenigen längsten zusammenhängenden Abfolge von Zeichen, das bzw. die sich rechts von dem Zeichen bzw. der längsten zusammenhängenden Abfolge von Zeichen befinden, das bzw. die in Schritt (ii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten links befindet;

(iv) Ausschließen jedes Zeichens und/oder jeder längsten zusammenhängenden Abfolge von Zeichen, das bzw. die im Schritt (iii) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(v)

- Auswählen desjenigen Zeichens oder derjenigen längsten zusammenhängenden Abfolge von Zeichen, das bzw. die sich in der zusammenhängenden Abfolge von Zeichen unmittelbar links von dem jeweiligen Zeichen bzw. der jeweiligen längsten zusammenhängenden Abfolge von Zeichen befinden, das bzw. die in vorstehendem Schritt ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet; und
- Auswählen desjenigen Zeichens oder derjenigen längsten zusammenhängenden Abfolge von Zeichen, das bzw. die sich in der zusammenhängenden Abfolge von Zeichen unmittelbar rechts von dem jeweiligen Zeichen bzw. der jeweiligen längsten zusammenhängenden Abfolge von Zeichen befinden, das bzw. die in vorstehendem Schritt ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten links befindet;

(vi) Ausschließen jedes Zeichens und/oder jeder längsten zusammenhängenden Abfolge von Zeichen, das bzw. die in Schritt (v) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(vii) Wiederholen der Schritte (v) und (vi), bis kein Zeichen oder keine längste zusammenhängende Abfolge von Zeichen mehr ausgewählt wird, das bzw. die in der ersten Liste von Zeichen und in der zweiten Liste von Zeichen gleich ist;
(viii) Addieren

- der Anzahl von Zeichen in der ersten Liste von Zeichen, die in jedem der Schritte (i) bis (vii) ausgeschlossen wurden; und
- der Anzahl von Zeichen in der zweiten Liste von Zeichen, die in jedem der Schritte (i) bis (vii) ausgeschlossen wurden,

um die Gesamtanzahl der Zeichen, Cc, in der ersten und der zweiten Liste von Zeichen zu erhalten, die gleich sind wie in der zweiten bzw. in der ersten Liste von Zeichen;
(ix) Addieren von

- $C_c$; und
- der Anzahl der Zeichen in der ersten Liste von Zeichen, die sich zwischen den Zeichen und/oder längsten zusammenhängenden Abfolgen von Zeichen befinden, die aus der ersten Liste von Zeichen ausgeschlossen wurden und die in keinem der Schritte (i) bis (vii) des Schrittes (c) ausgeschlossen wurden; und
- der Anzahl der Zeichen in der zweiten Liste von Zeichen, die sich zwischen den Zeichen und/oder längsten zusammenhängenden Abfolgen von Zeichen befinden, die aus der zweiten Liste von Zeichen ausgeschlossen wurden und die in keinem der Schritte (i) bis (vii) des Schrittes (c) ausgeschlossen wurden,

um die Gesamtanzahl von Zeichen, Ct, in der ersten und der zweiten Liste von Zeichen zu erhalten; und
(x) Berechnen des DS gemäß der nachstehenden Formel:

$$DS = C_c / C_t$$

oder auf folgendem Wege:

(xi) Auswählen des Zeichens oder der längsten zusammenhängenden Abfolge von Zeichen, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet;
(xii) Ausschließen des Zeichens oder der längsten zusammenhängenden Abfolge von Zeichen, das bzw. die in Schritt (xi) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(xiii)

- Auswählen desjenigen Zeichens oder derjenigen längsten zusammenhängenden Abfolge von Zeichen, das bzw. die sich links von dem Zeichen bzw. der längsten zusammenhängenden Abfolge von Zeichen befinden, das bzw. die in Schritt (xii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten rechts befindet; und
- Auswählen desjenigen Zeichens oder derjenigen längsten zusammenhängenden Abfolge von Zeichen, das bzw. die sich rechts von dem Zeichen bzw. der längsten zusammenhängenden Abfolge von Zeichen befinden, das bzw. die in Schritt (xii) ausgeschlossen wurde, die in der ersten Liste von Zeichen und der zweiten Liste von Zeichen gleich sind, wobei, wenn zwei oder mehr Zeichen oder zwei oder mehr längste zusammenhängende Abfolgen mit derselben Länge ausgewählt sind, nur das Zeichen oder die längste zusammenhängende Abfolge von Zeichen ausgewählt wird, das bzw. die sich in der Liste von Zeichen am weitesten links befindet;

(xiv) Ausschließen jedes Zeichens und/oder jeder längsten zusammenhängenden Abfolge von Zeichen, das bzw. die in Schritt (xiii) ausgewählt wurde, aus den nachfolgenden Schritten des Auswählens eines Zeichens oder einer längsten zusammenhängenden Abfolge von Zeichen, das bzw. die in der ersten Liste von Zeichen und in der zweiten Liste von Zeichen gleich ist;
(xv) Wiederholen der Schritte (xiii) und (xiv), bis kein Zeichen oder keine längste zusammenhängende Abfolge von Zeichen mehr ausgewählt wird, das bzw. die in der ersten Liste von Zeichen und in der zweiten Liste von Zeichen gleich ist;
(xvi) Addieren

- der Anzahl von Zeichen in der ersten Liste von Zeichen, die in jedem der Schritte (xi) bis (xv) ausgeschlossen wurden; und
- der Anzahl von Zeichen in der zweiten Liste von Zeichen, die in jedem der Schritte (xi) bis (xv) ausgeschlossen wurden, um die Gesamtanzahl der Zeichen, Cc, in der ersten und der zweiten Liste von Zeichen zu erhalten, die gleich sind wie in der zweiten bzw. in der ersten Liste von Zeichen;

(xvii) Addieren von

- $C_c$; und
- der Anzahl der Zeichen in der ersten Liste von Zeichen, die sich zwischen den Zeichen und/oder längsten zusammenhängenden Abfolgen von Zeichen befinden, die aus der ersten Liste von Zeichen ausgeschlossen wurden und die in keinem der Schritte (xi) bis (xv) des Schrittes (c) ausgeschlossen wurden; und
- der Anzahl der Zeichen in der zweiten Liste von Zeichen, die sich zwischen den Zeichen und/oder längsten zusammenhängenden Abfolgen von Zeichen befinden, die aus der zweiten Liste von Zeichen ausgeschlossen wurden und die in keinem der Schritte (xi) bis (xv) des Schrittes (c) ausgeschlossen wurden,

um die Gesamtanzahl von Zeichen, Ct, in der ersten und der zweiten Liste von Zeichen zu erhalten; und
(xviii) Berechnen des DS gemäß der nachstehenden Formel:

$$DS = C_c / C_t$$

(d) Mittel oder Befehle zum Auswählen des DS mit dem höchsten Wert, $DS_{HV}$, für jede im Schritt (a) erhaltene erste Liste von Zeichen;
(e) Mittel oder Befehle zum Addieren der Anzahl der ersten Listen von Zeichen mit einem $DS_{HV}$, der größer ist als ein Grenzwert T, um die Gesamtanzahl der ersten Listen von Zeichen, $L_c$, zu erhalten, die gleich sind wie eine zweite Liste von Zeichen;
(f) Mittel oder Befehle zum Addieren von

- $L_c$; und
- der Anzahl der ersten Listen von Zeichen ohne $DS_{HV}$, der größer ist als T,

um die Gesamtanzahl von ersten Listen von Zeichen, $L_t$, zu erhalten; und
(g) Mittel und Befehle zum Berechnen des Grads der minimalen Resterkrankung (MRD) gemäß der nachstehenden Formel:

$$MRD = L_c \times (D / k) / L_t^2 ;$$

und wobei die Mittel der Schritte (a) bis (g) in der Lage sind, die MRD zu berechnen, oder Befehle zum Berechnen der MRD bereitstellen, ohne dass dazu auf externe Datenbanken zugegriffen werden muss; und wobei die Mittel zumindest einen Rechner oder zumindest einen Schaltkreis oder zumindest einen integrierten Schaltkreis oder zumindest einen Chip oder zumindest einen Mikrochip umfassen; und wobei die Befehle von einem menschlichen Anwender oder zumindest einem Rechner oder zumindest einem Schaltkreis oder zumindest einem integrierten Schaltkreis oder zumindest einem Chip oder zumindest einem Mikrochip ausgeführt werden können.

**9.** Set nach Anspruch 8, wobei:

- jedes Zeichen in jeder der ersten und der zweiten Liste von Zeichen, das in Schritt (a) bzw. (b) erhalten wurde, einen Buchstaben umfasst, der einer Zahl oder einem Symbol zugeordnet ist, wobei die Zahl oder das Symbol für Qualität (*Q*) steht, wobei die Qualität (*Q*) eine ganzzahlige Funktion der Wahrscheinlichkeit ist, dass der Buchstabe, der für ein Nukleotid steht, das an der entsprechenden Position in der Nukleotidsequenz identifiziert wurde, falsch ist, und wobei der Buchstabe für das Nukleotid steht, das an der entsprechenden Position in der Nukleotidsequenz mit der höchsten Qualität (*Q*) identifiziert wurde; und
- ein Zeichen in der ersten Liste von Zeichen in Schritt (c) als gleich wie ein Zeichen in der zweiten Liste von Zeichen ermittelt wird, wenn der Buchstabe mit der höchsten Qualität in der ersten und der zweiten Liste von Zeichen gleich ist.

**10.** Set nach Anspruch 9, wobei ein Zeichen in der ersten Liste von Zeichen als gleich wie ein Zeichen in der zweiten Liste von Zeichen ermittelt wird, wenn der Buchstabe mit der höchsten Qualität (*Q*) in der ersten und der zweiten Liste von Zeichen gleich ist und die Qualität des Buchstabens in der ersten Liste von Zeichen innerhalb von 1 Prozent der Qualität des Buchstabens in der zweiten Liste von Zeichen liegt.

**11.** Set nach einem der Ansprüche 8 bis 10, wobei eine MRD von 1 zu 100 % auf eine Erkrankung bei dem Individuum hindeutet und eine MRD von 0 zu 0 % auf eine Erkrankung bei dem Individuum hindeutet.

**12.** Set nach einem der Ansprüche 8 bis 11, wobei die Erkrankung aus Krebs oder Leukämie ausgewählt ist.

**13.** Set nach Anspruch 12, wobei die Erkrankung aus akuter lymphoblastischer Leukämie, akuter myeloischer Leukämie, chronischer lymphatischer Leukämie, chronischer myeloischer Leukämie, follikulärem Lymphom, Mantelzelllymphom, multiplem Myelom, Brustkrebs oder Neuroblastom ausgewählt ist.

**14.** Set nach Anspruch 8, wobei die in den Schritten (a) und/oder (b) verwendeten Mittel zum Sequenzieren aus Multiplex- und/oder Hochdurchsatz-Nukleotidsequenzierung; massiv-paralleler Signatursequenzierung (MPSS) und massiv-paralleler Sequenzierung unter Verwendung von Emulsions-PCR ausgewählt sind.

**15.** Verwendung des Verfahrens nach einem der Ansprüche 1 bis 7 oder des Sets nach einem der Ansprüche 8 bis 14 zum Quantifizieren des Grads der minimalen Resterkrankung (MRD) bei einem Individuum, das wegen dieser Erkrankung behandelt wurde.

## Revendications

**1.** Procédé pour quantifier le niveau de maladie résiduelle (MRD) chez un sujet traité pour ladite maladie, qui comprend :

(a)

- l'amplification par réaction en chaîne par polymérase en utilisant des amorces, d'au moins une séquence nucléotidique comprise dans une quantité D d'ADN génomique d'un échantillon biologique obtenu sur ledit sujet après qu'il a été traité pour ladite maladie, l'ADN génomique ayant un poids moyen k par cellule diploïde dudit échantillon biologique ; et
- le séquençage de ladite au moins une séquence nucléotidique pour obtenir au moins une première liste de caractères à lire de gauche à droite ;

(b)

- l'amplification par réaction en chaîne par polymérase en utilisant les mêmes amorces qu'à l'étape (a), au moins une séquence nucléotidique dans un échantillon biologique obtenu sur ledit sujet avant qu'il soit traité pour ladite maladie ; et
- le séquençage de ladite au moins une séquence nucléotidique pour obtenir au moins une seconde liste de caractères à lire de gauche à droite ;

(c) la détermination, pour chaque première liste de caractères obtenus à l'étape (a), du degré de similitude avec chaque seconde liste de caractères obtenus à l'étape (b), le degré de similitude, DS, d'une première liste de caractères obtenus à l'étape (a) avec une seconde liste de caractères obtenus à l'étape (b), est déterminé soit :

(i) en sélectionnant le caractère ou la séquence de caractères continue la plus longue qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ;
(ii) en excluant le caractère ou la séquence de caractères continue la plus longue sélectionné(e) à l'étape (i) des étapes ultérieures de sélection d'un caractère ou de la séquence de caractères continue la plus longue qui est identique dans la première liste de caractères et la seconde liste de caractères ;
(iii)

- en sélectionnant le caractère ou la séquence de caractères continue la plus longue qui est situé(e) à la gauche du caractère ou de la séquence de caractères continue la plus longue exclu(e) à l'étape (ii) qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ; et
- en sélectionnant le caractère ou la séquence de caractères continue la plus longue qui est situé(e) à la droite du caractère ou de la séquence de caractères continue la plus longue exclu(e) à l'étape (ii) qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à gauche dans les listes de caractères est sélectionné(e) ;

(iv) en excluant chaque caractère et/ou chaque séquence de caractères continue la plus longue sélectionné(e) à l'étape (iii) des étapes ultérieures de sélection d'un caractère ou de la séquence de caractères continue la plus longue qui est identique dans la première liste de caractères et la seconde liste de caractères ;
(v)

- en sélectionnant le caractère ou la séquence de caractères continue la plus longue qui est situé(e) dans la séquence de caractères continue immédiatement à gauche de chaque caractère ou de chaque séquence de caractères continue la plus longue exclu(e) à l'étape précédente qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ; et
- en sélectionnant le caractère ou la séquence de caractères continue la plus longue qui est situé(e) dans la séquence de caractères continue immédiatement à la droite de chaque caractère ou de chaque séquence de caractères continue la plus longue exclu(e) à l'étape précédente qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à gauche dans les listes de caractères est sélectionné(e) ;

(vi) en excluant chaque caractère et/ou chaque séquence de caractères continue la plus longue sélectionné(e) à l'étape (v) des étapes ultérieures de sélection d'un caractère ou de la séquence de caractères continue la plus longue qui est identique dans la première liste de caractères et la seconde liste de caractères ;
(vii) en répétant les étapes (v) et (vi) jusqu'à ce que qu'aucun caractère ou aucune séquence de caractères continue la plus longue identique dans la première liste de caractères et la seconde liste de caractères soit sélectionné(e) ;
(viii) en additionnant

- le nombre de caractères dans la première liste de caractères qui ont été exclus dans n'importe laquelle des étapes (i) à (vii) ; et
- le nombre de caractères dans la seconde liste de caractères qui ont été exclus dans n'importe laquelle des étapes (i) à (vii) pour obtenir le nombre total de caractères, $C_c$, dans les première et seconde liste de caractères qui sont identiques à ceux des seconde et première liste de caractères respectivement ;

(ix) en additionnant

- $C_c$ ; et
- le nombre de caractères dans la première liste de caractères qui sont situés entre les caractères et/ou les séquences de caractères continues les plus longues qui ont été exclus de la première liste de caractères et qui n'ont pas été exclus à n'importe laquelle des étapes (i) à (vii) de l'étape (c) ; et
- le nombre de caractères dans la seconde liste de caractères qui sont situés entre les caractères et/ou les séquences de caractères continues les plus longues qui ont été exclus de la seconde liste de caractères et qui n'ont pas été exclus à n'importe laquelle des étapes (i) à (vii) de l'étape (c),

pour obtenir le nombre total de caractères, $C_t$, dans les première et seconde listes de caractères ; et
(x) en calculant le DS selon la formule suivante :

$$DS = C_c / C_t$$

ou :

(xi) en sélectionnant le caractère ou la séquence de caractères continue la plus longue qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ;
(xii) en excluant le caractère ou la séquence de caractères continue la plus longue sélectionné(e) à l'étape (xi) des étapes ultérieures de sélection d'un caractère ou de la séquence de caractères continue la plus longue qui est identique dans la première liste de caractères et la seconde liste de caractères ;
(xiii)

- en sélectionnant le caractère ou la séquence de caractères continue la plus longue qui est situé(e) à la gauche du caractère ou de la séquence de caractères continue la plus longue exclu(e) à l'étape (xii) qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ; et
- en sélectionnant le caractère ou la séquence de caractères continue la plus longue qui est situé(e) à la droite du caractère ou de la séquence de caractères continue la plus longue exclu(e) à l'étape (xii) qui sont identiques dans la première liste de caractères et dans la seconde liste de caractères, où lorsque deux caractères ou plus ou deux séquences continues les plus longues ou plus de même longueur sont sélectionnés, seul(e) le caractère ou la séquence de caractères continue la plus longue le plus à gauche dans les listes de caractères est sélectionné(e) ;

(xiv) en excluant chaque caractère et/ou chaque séquence de caractères continue la plus longue sélectionné(e) à l'étape (xiii) des étapes ultérieures de sélection d'un caractère ou de la séquence de caractères continue la plus longue qui est identique dans la première liste de caractères et la seconde liste de caractères ;
(xv) en répétant les étapes (xiii) et (xiv) jusqu'à ce que qu'aucun caractère ou aucune séquence de caractères continue la plus longue identique dans la première liste de caractères et la seconde liste de caractères soit sélectionné(e) ;
(xvi) en additionnant

- le nombre de caractères dans la première liste de caractères qui ont été exclus dans n'importe laquelle des étapes (xi) à (xv) ; et
- le nombre de caractères dans la seconde liste de caractères qui ont été exclus dans n'importe laquelle des étapes (xi) à (xv) pour obtenir le nombre total de caractères, $C_c$, dans les première et seconde listes de caractères qui sont identiques à ceux des seconde et première listes de caractères respectivement ;

(xvii) en additionnant

- $C_c$ ; et
- le nombre de caractères dans la première liste de caractères qui sont situés entre les caractères et/ou les séquences de caractères continues les plus longues qui ont été exclus de la première liste de caractères et qui n'ont pas été exclus à n'importe laquelle des étapes (xi) à (xv) de l'étape (c) ; et
- le nombre de caractères dans la seconde liste de caractères qui sont situés entre les caractères et/ou les séquences de caractères continues les plus longues qui ont été exclus de la seconde liste de caractères et qui n'ont pas été exclus à n'importe laquelle des étapes (xi) à (xv) de l'étape (c),

pour obtenir le nombre total de caractères, $C_t$, dans les première et seconde listes de caractères ; et
(xviii) en calculant le DS selon la formule suivante :

$$DS = C_c / C_t$$

(d) en sélectionnant, pour chaque première liste de caractères obtenus à l'étape (a), le DS de la plus grande valeur, $DS_{HV}$;
(e) en additionnant le nombre des premières listes de caractères dont la valeur $DS_{HV}$ est supérieure à une valeur de seuil, T, afin d'obtenir le nombre total des premières listes de caractères, $L_c$, qui sont identiques à ceux d'une seconde liste de caractères ;
(f) en additionnant

- $L_c$ ; et
- le nombre des premières listes de caractères qui n'ont pas de valeur $DS_{HV}$ supérieure à T,

pour obtenir le nombre total des premières listes de caractères, $L_t$; et
(g) en calculant le niveau de maladie résiduelle (MRD) selon la formule suivante :

$$MRD = L_c \times (D / k) / L_t^2.$$

**2.** Procédé selon la revendication 1, dans lequel

- chaque caractère dans chacune des première et seconde listes de caractères respectivement obtenus aux étapes (a) et (b) comprennent une lettre associée à un numéro ou à un symbole, ledit numéro ou symbole représentant la qualité (*Q*), la qualité (*Q*) étant une cartographie d'entiers de la probabilité que la lettre qui représente un nucléotide qui est identifié dans la position correspondante dans la séquence nucléotidique soit incorrecte, et ladite lettre représentant le nucléotide qui est identifié dans la position correspondante dans la séquence nucléotidique ayant la meilleure qualité (*Q*) ; et
- un caractère dans la première liste de caractères est déterminé à l'étape (c) comme étant identique à un caractère dans la seconde liste de caractères, lorsque la lettre ayant la meilleure qualité est identique dans les première et seconde listes de caractères.

**3.** Procédé selon la revendication 2, dans lequel un caractère dans la première liste de caractères est déterminé à l'étape (c) comme étant identique à un caractère dans la seconde liste de caractères, lorsque la lettre ayant la meilleure qualité (*Q*) est identique dans les première et seconde listes de caractères et la qualité de la lettre dans la première liste de caractères est comprise dans 1 % de la qualité de la lettre dans la seconde liste de caractères.

**4.** Procédé selon l'une quelconque des revendications 1 à 3, dans lequel le séquençage est le séquençage massif en parallèle.

**5.** Procédé selon l'une quelconque des revendications précédentes 1 à 4, dans lequel une MRD de 1 indique l'existence à 100 % de la maladie chez ledit sujet et une MRD de 0 indique l'existence à 0 % de la maladie chez ledit sujet.

**6.** Procédé selon l'une quelconque des revendications 1 à 5, dans lequel la maladie est sélectionnée parmi le cancer ou la leucémie.

**7.** Procédé selon la revendication 6, dans lequel la maladie est sélectionnée parmi la leucémie lymphoblastique aiguë, la leucémie myéloïde aiguë, la leucémie lymphocytaire chronique, la leucémie myéloïde chronique, le lymphome folliculaire, le lymphome du manteau, le myélome multiple, le cancer du sein ou le neuroblastome.

**8.** Kit pour quantifier le niveau de maladie résiduelle (MRD) chez un sujet traité pour ladite maladie, qui comprend :

(a)

- des moyens pour amplifier dans un instrument de PCR, par réaction en chaîne par polymérase, au moins une séquence nucléotidique comprise dans une quantité, D, d'ADN génomique d'un échantillon biologique prélevé sur ledit sujet après qu'il a été traité pour ladite maladie, l'ADN génomique ayant un poids moyen, k, par cellule diploïde dudit échantillon biologique, lesdits moyens comprenant des amorces sens spécifiques de locus et des amorces antisens spécifiques de locus, pour identifier un variant spécifique d'une séquence nucléotidique présente dans l'échantillon biologique, ledit un variant ou l'absence dudit un variant indiquant la maladie ; et
- des moyens pour le séquençage de ladite au moins une séquence nucléotidique pour obtenir au moins une première liste de caractères à lire de gauche à droite, le séquençage comprenant une plate-forme de séquençage massif en parallèle ;

(b)

- des moyens pour l'amplification, par réaction en chaîne par polymérase dans un instrument de PCR, en utilisant les mêmes amorces spécifiques de locus qu'à l'étape (a), au moins une séquence nucléotidique dans un échantillon biologique obtenu sur ledit sujet avant qu'il soit traité pour ladite maladie ; et
- des moyens pour le séquençage de ladite au moins une séquence nucléotidique pour obtenir au moins une seconde liste de caractères à lire de gauche à droite ; le séquençage comprenant une plate-forme de séquençage massif en parallèle ;

(c) des moyens pour la détermination, pour chaque première liste de caractères obtenus à l'étape (a), du degré de similitude avec chaque seconde liste de caractères obtenus à l'étape (b), lesdits moyens comprenant eu préférence au moins un ordinateur, au moins un circuit, au moins un circuit intégré, au moins une puce ou au moins un microprocesseur et le degré de similitude, DS, d'une première liste de caractères obtenus à l'étape (a) avec une seconde liste de caractères obtenus à l'étape (b), est déterminé soit :

(i) en sélectionnant le caractère ou la séquence de caractères continue la plus longue qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ;
(ii) en excluant le caractère ou la séquence de caractères continue la plus longue sélectionné(e) à l'étape (i) des étapes ultérieures de sélection d'un caractère ou de la séquence de caractères continue la plus longue qui est identique dans la première liste de caractères et la seconde liste de caractères ;
(iii)

- en sélectionnant le caractère ou la séquence de caractères continue la plus longue qui est situé(e) à la gauche du caractère ou de la séquence de caractères continue la plus longue exclu(e) à l'étape (ii) qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ; et
- en sélectionnant le caractère ou la séquence de caractères continue la plus longue qui est situé(e) à la droite du caractère ou de la séquence de caractères continue la plus longue exclu(e) à l'étape (ii) qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à gauche dans les listes de caractères est sélectionné(e) ;

(iv) en excluant chaque caractère et/ou chaque séquence de caractères continue la plus longue sélectionné(e) à l'étape (iii) des étapes ultérieures de sélection d'un caractère ou de la séquence de caractères continue la plus longue qui est identique dans la première liste de caractères et la seconde liste de caractères ;
(v)

- en sélectionnant le caractère ou la séquence de caractères continue la plus longue qui est situé(e) dans la séquence de caractères continue immédiatement à gauche de chaque caractère ou de chaque séquence de caractères continue la plus longue exclu(e) à l'étape précédente qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ; et
- en sélectionnant le caractère ou la séquence de caractères continue la plus longue qui est situé(e) dans la séquence de caractères immédiatement à la droite de chaque caractère ou de chaque séquence de caractères continue la plus longue exclu(e) à l'étape précédente qui sont identiques dans la première liste de caractères et la seconde liste de caractères, lorsque deux caractères ou plus ou deux séquences continues les plus longues ou plus de même longueur sont sélectionnés, seul(e) le caractère ou la séquence de caractères continue la plus longue le plus à gauche dans les listes de caractères est sélectionné(e) ;

(vi) en excluant chaque caractère et/ou chaque séquence de caractères continue la plus longue sélectionné(e) à l'étape (v) des étapes ultérieures de sélection d'un caractère ou de la séquence de caractères continue la plus longue qui est identique dans la première liste de caractères et la seconde liste de caractères ;
(vii) en répétant les étapes (v) et (vi) jusqu'à ce que qu'aucun caractère ou aucune séquence de caractères continue la plus longue identique dans la première liste de caractères et la seconde liste de caractères soit sélectionné(e) ;
(viii) en additionnant

- le nombre de caractères dans la première liste de caractères qui ont été exclus dans n'importe laquelle des étapes (i) à (vii) ; et
- le nombre de caractères dans la seconde liste de caractères qui ont été exclus dans n'importe laquelle des étapes (i) à (vii) pour obtenir le nombre total de caractères, $C_c$, dans les première et seconde listes de caractères qui sont identiques à ceux des seconde et première listes de caractères respectivement ;

(ix) en additionnant

- $C_c$ ; et
- le nombre de caractères dans la première liste de caractères qui sont situés entre les caractères et/ou les séquences de caractères continues les plus longues qui ont été exclus de la première liste de caractères et qui n'ont pas été exclus à n'importe laquelle des étapes (i) à (vii) de l'étape (c) ; et
- le nombre de caractères dans la seconde liste de caractères qui sont situés entre les caractères et/ou les séquences de caractères continues les plus longues qui ont été exclus de la seconde liste de caractères et qui n'ont pas été exclus à n'importe laquelle des étapes (i) à (vii) de l'étape (c),

pour obtenir le nombre total de caractères, $C_t$, dans les première et seconde listes de caractères ; et
(x) en calculant le DS selon la formule suivante :

$$DS = C_c / C_t$$

ou :

(xi) en sélectionnant le caractère ou la séquence de caractères continue la plus longue qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ;
(xii) en excluant le caractère ou la séquence de caractères continue la plus longue sélectionné(e) à l'étape (xi) des étapes ultérieures de sélection d'un caractère ou de la séquence de caractères continue la plus longue qui est identique dans la première liste de caractères et la seconde liste de caractères ;
(xiii)

- en sélectionnant le caractère ou la séquence de caractères continue la plus longue qui est situé(e) à la gauche du caractère ou de la séquence de caractères continue la plus longue exclu(e) à l'étape (xii) qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à droite dans les listes de caractères est sélectionné(e) ; et
- en sélectionnant le caractère ou la séquence de caractères continue la plus longue qui est situé(e) à la droite du caractère ou de la séquence de caractères continue la plus longue exclu(e) à l'étape (xii) qui sont identiques dans la première liste de caractères et la seconde liste de caractères, où lorsque deux caractères ou plus ou deux séquences continues les plus longues ou plus de même longueur sont sélectionnés, seul(e) le caractère ou la séquence de caractères continue la plus longue le plus à gauche dans les listes de caractères est sélectionné(e) ;

(xiv) en excluant chaque caractère et/ou chaque séquence de caractères continue la plus longue sélectionné(e) à l'étape (xiii) des étapes ultérieures de sélection d'un caractère ou de la séquence de caractères continue la plus longue qui est identique dans la première liste de caractères et la seconde liste de caractères ;
(xv) en répétant les étapes (xiii) et (xiv) jusqu'à ce que qu'aucun caractère ou aucune séquence de caractères continue la plus longue identique dans la première liste de caractères et la seconde liste de caractères soit sélectionné(e) ;
(xvi) en additionnant

- le nombre de caractères dans la première liste de caractères qui ont été exclus à n'importe laquelle des étapes (xi) à (xv) ; et
- le nombre de caractères dans la seconde liste de caractères qui ont été exclus à n'importe laquelle des étapes (xi) à (xv) pour obtenir le nombre total de caractères, $C_c$, dans les première et seconde listes de caractères qui sont identiques à ceux des seconde et première listes de caractères respectivement ;

(xvii) en additionnant

- $C_c$ ; et
- le nombre de caractères dans la première liste de caractères qui sont situés entre les caractères et/ou les séquences de caractères continues les plus longues qui ont été exclus de la première liste de caractères et qui n'ont pas été exclus à n'importe laquelle des étapes (xi) à (xv) de l'étape (c) ; et
- le nombre de caractères dans la seconde liste de caractères qui sont situés entre les caractères et/ou les séquences de caractères continues les plus longues qui ont été exclus de la seconde liste de caractères et qui n'ont pas été exclus à n'importe laquelle des étapes (xi) à (xv) de l'étape (c),

pour obtenir le nombre total de caractères, $C_t$, dans les première et seconde listes de caractères ; et
(xviii) en calculant le DS selon la formule suivante :

$$DS = C_c / C_t$$

(d) des moyens ou des instructions pour sélectionner, pour chaque première liste de caractères obtenus à l'étape (a), le DS de la plus grande valeur, $DS_{HV}$;
(e) des moyens ou des instructions pour additionner le nombre des premières listes de caractères dont la valeur $DS_{HV}$ est supérieure à une valeur de seuil, T, afin d'obtenir le nombre total des premières listes de caractères, $L_c$, qui sont identiques à ceux d'une seconde liste de caractères ;
(f) des moyens ou des instructions pour additionner

- $L_c$ ; et
- le nombre des premières listes de caractères qui n'ont pas de valeur $DS_{HV}$ supérieure à T,

pour obtenir le nombre total des premières listes de caractères, $L_t$; et
(g) des moyens et des instructions pour calculer le stade de la maladie résiduelle (MRD) selon la formule suivante :

$$MRD = L_c \text{ x } (D / k) / L_t^2 ;$$

et dans lequel lesdits moyens des étapes (a) à (g) sont capables de calculer la MRD ou fournissent des instructions pour calculer la MRD sans avoir besoin d'accéder à des bases de données externes ; et dans lequel lesdits moyens comprennent au moins un ordinateur, ou au moins un circuit, ou au moins un circuit intégré, ou au moins une puce ou au moins un microprocesseur ; et dans lequel lesdites instructions peuvent être exécutées par un opérateur humain, ou au moins un ordinateur, ou au moins un circuit, ou au moins un circuit intégré, ou au moins une puce, ou au moins un microprocesseur.

**9.** Kit selon la revendication 8, dans lequel

- chaque caractère dans chacune des première et seconde listes de caractères respectivement obtenus aux étapes (a) et (b) comprennent une lettre associée à un numéro ou à un symbole, ledit numéro ou symbole représentant la qualité (*Q*), la qualité (*Q*) étant une cartographie d'entiers de la probabilité que la lettre qui représente un nucléotide qui est identifié dans la position correspondante dans la séquence nucléotidique soit incorrecte, et ladite lettre représentant le nucléotide qui est identifié dans la position correspondante dans la séquence nucléotidique ayant la meilleure qualité (*Q*) ; et
- un caractère dans la première liste de caractères est déterminé à l'étape (c) comme étant identique à un caractère dans la seconde liste de caractères, lorsque la lettre ayant la meilleure qualité est identique dans les première et seconde listes de caractères.

**10.** Kit selon la revendication 9, dans lequel un caractère dans la première liste de caractères est déterminé comme étant identique à un caractère dans la seconde liste de caractères, lorsque la lettre ayant la meilleure qualité (*Q*) est identique dans les première et seconde listes de caractères et la qualité de la lettre dans la première liste de caractères est comprise dans 1 % de la qualité de la lettre dans la seconde liste de caractères.

**11.** T Kit selon l'une quelconque des revendications précédentes 8 à 10, dans lequel une MRD de 1 indique l'existence à 100 % de la maladie chez ledit sujet et une MRD de 0 indique l'existence à 0 % de la maladie chez ledit sujet.

**12.** Kit selon l'une quelconque des revendications 8 à 11, dans lequel la maladie est sélectionnée parmi le cancer ou la leucémie

**13.** Kit selon la revendication 12, dans lequel la maladie est sélectionnée parmi la leucémie lymphoblastique aiguë, la leucémie myéloïde aiguë, la leucémie lymphocytaire chronique, la leucémie myéloïde chronique, le lymphome folliculaire, le lymphome du manteau, le myélome multiple, le cancer du sein ou le neuroblastome.

**14.** Kit selon la revendication 8, dans lequel les moyens de séquençage utilisés aux étapes (a) et/ou (b) sont sélectionnés parmi le séquençage multiplex et/ou haut débit, le séquençage massif des signatures en parallèle (MPSS) et le séquençage massif en parallèle utilisant une PCR en émulsion.

**15.** Utilisation du procédé selon l'une quelconque des revendications 1 à 7 ou kit selon l'une quelconque des revendications 8 à 14 pour quantifier le niveau de maladie résiduelle (MRD) chez un sujet qui est traité pour ladite maladie.

**Figure 1**

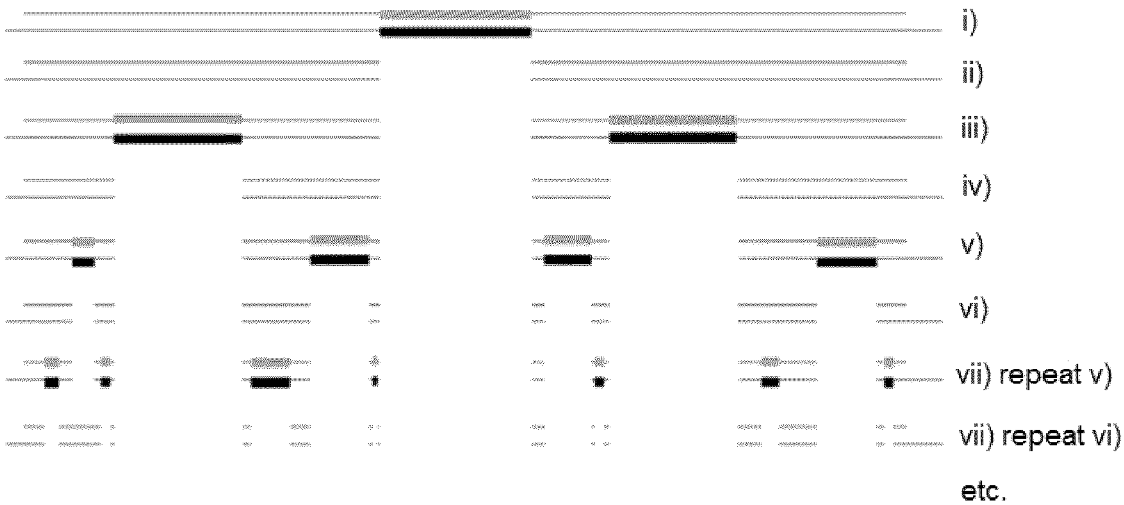

Figure 2

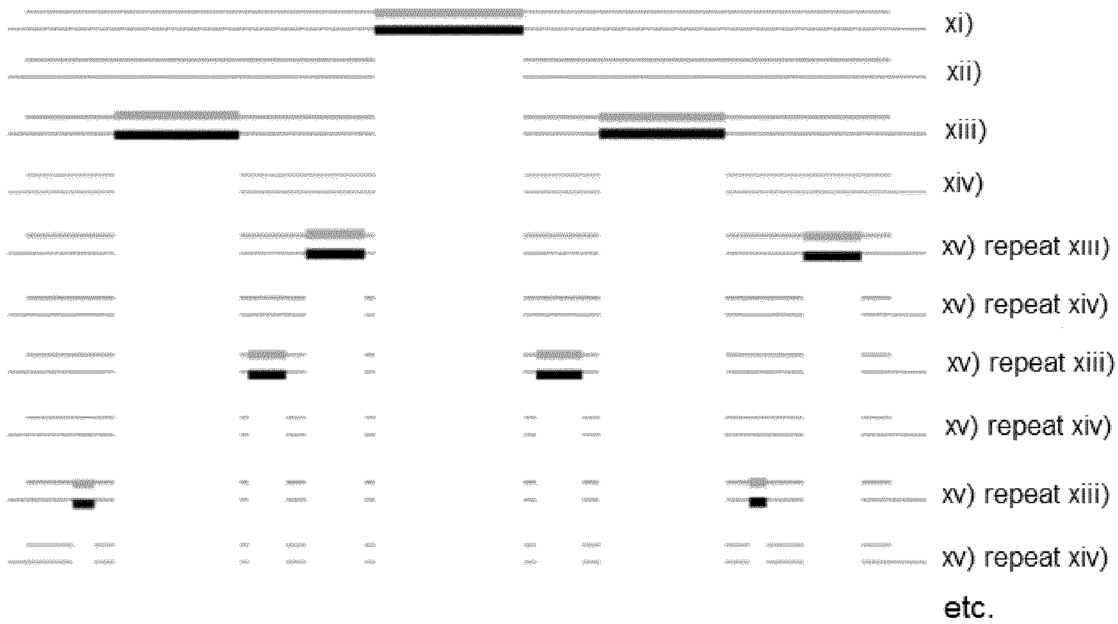
xi)
xii)
xiii)
xiv)
xv) repeat xiii)
xv) repeat xiv)
xv) repeat xiii)
xv) repeat xiv)
xv) repeat xiii)
xv) repeat xiv)
etc.

Figure 3

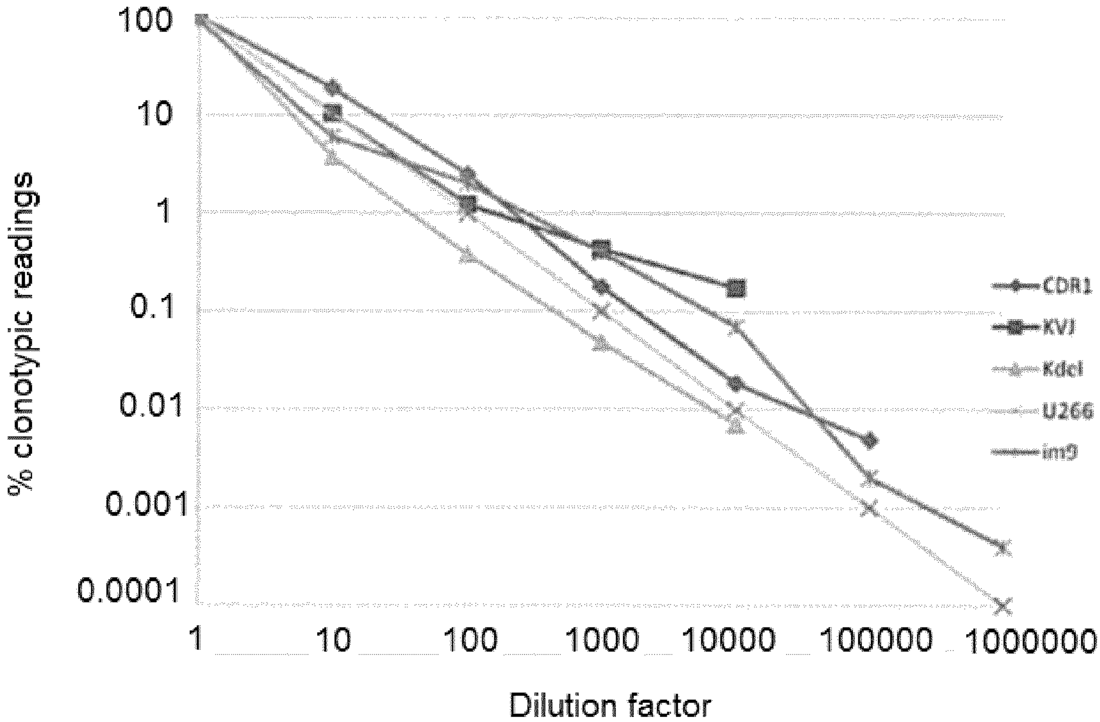
100
10
1
0.1
0.01
0.001
0.0001
% clonotypic readings
1
10
100
1000
10000
100000
1000000
Dilution factor
CDR1
KVJ
Kdel
U266
im9

Figure 4

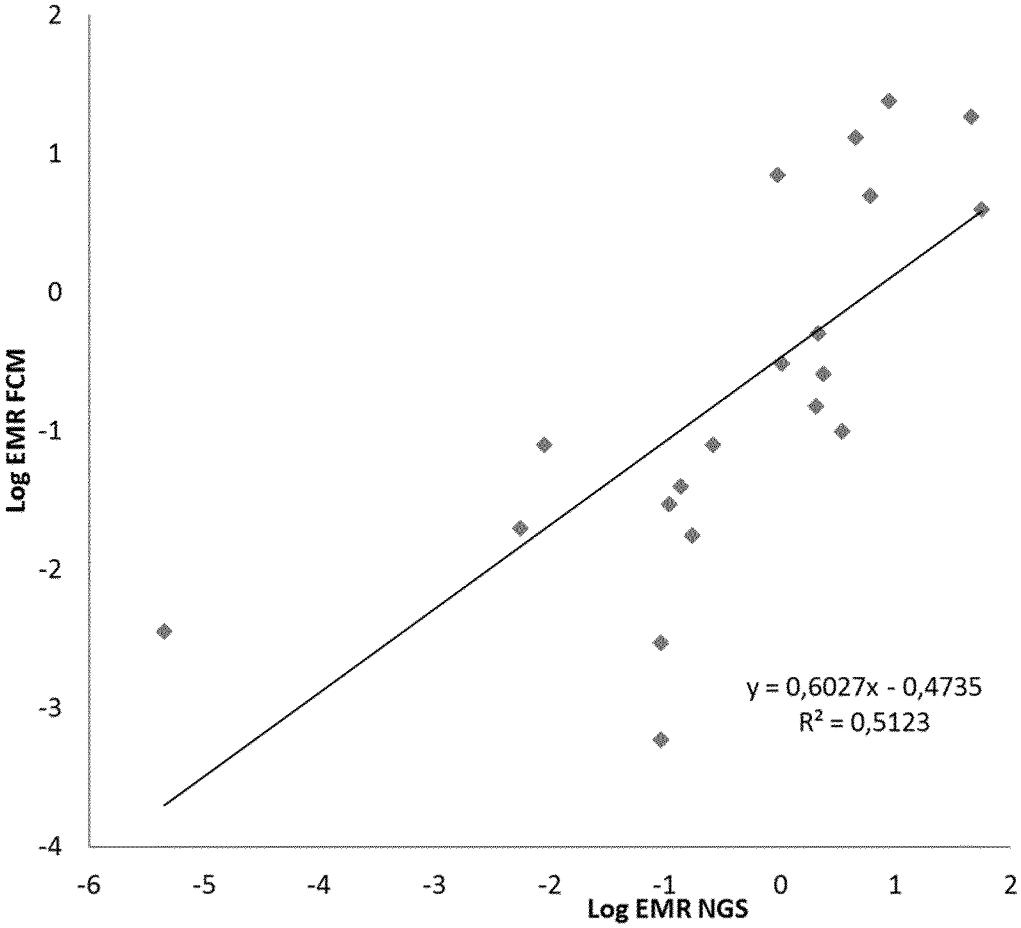
2
1
0
-1
-2
-3
-4
Log EMR FCM
-6
-5
-4
-3
-2
-1
0
1
2
Log EMR NGS
y = 0,6027x - 0,4735
R² = 0,5123

**Figure 5**

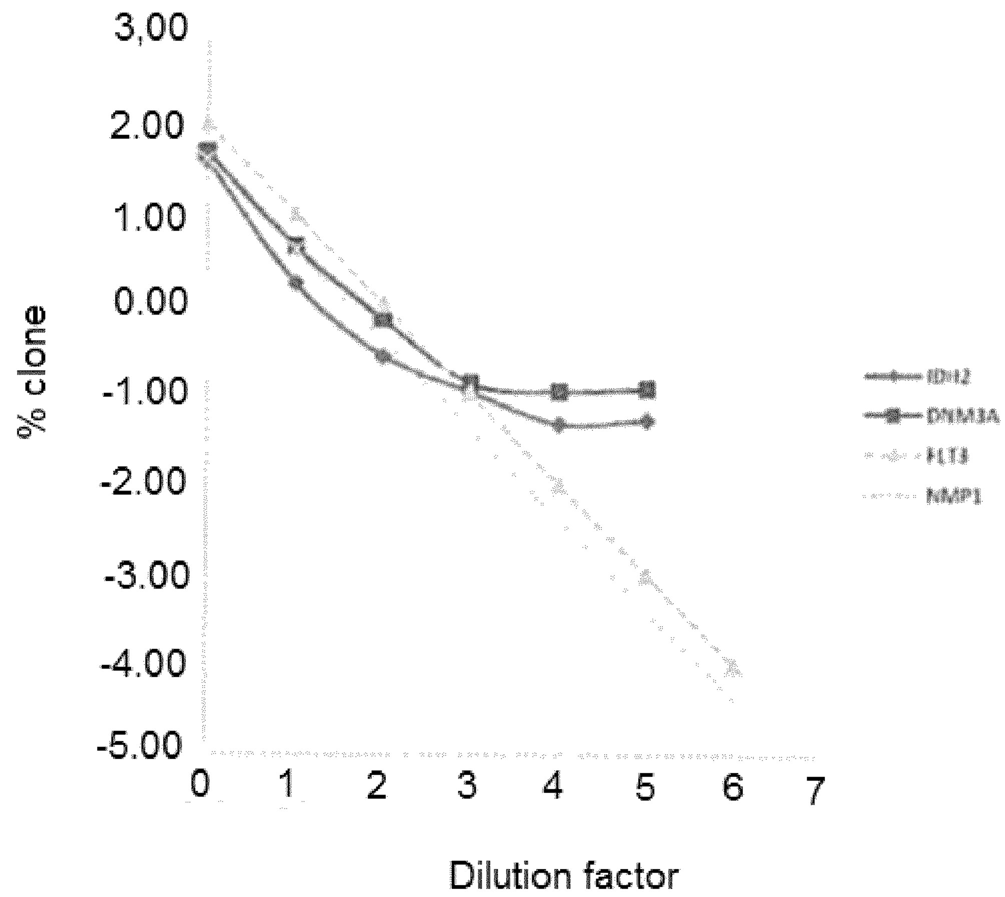
3,00
2.00
1.00
0.00
-1.00
-2.00
-3.00
-4.00
-5.00
% clone
0
1
2
3
4
5
6
7
Dilution factor
IDH2
DNM3A
FLT3
NMP1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004033728 A2
- US 7785783 B2
- US 8628927 B2
- WO 2013188471 A2


### Non-patent literature cited in the description


- **MARTINEZ-LOPEZ J. et al.** Prognostic value of deep sequencing method for minimal residual disease detection in multiple myeloma. *Blood,* 2014, vol. 123 (20), 3073-3079
- **VIJ R. et al.** Deep sequencing reveals myeloma cells in peripheral blood in majority of multiple myeloma patients. *J. Clin. Lymphoma Myeloma Leuk.,* 2013, vol. 14 (2), 131-139
- **LADETTO M. et al.** Next-generation sequencing and real-time quantitative PCR for minimal residual disease detection in B-cell disorders. *Leukemia,* 2014, vol. 28 (6), 1299-1307
- **FAHAM et al.** *Blood,* 2012, vol. 120 (26), 5173-5180
- **HAUWEL M. ; MATTHES T.** Minimal residual disease monitoring: the new standard for treatment evaluation of haematological malignancies?. *Swiss Med Wkly,* 2014, vol. 144, w13907

Sequence Listing

<110> Fundacion de Investigacion Hospital 12 de Octubre

<120> Method for quantifying the level of minimal residual disease in a subject

<130> EP-06895

<160> 38

<170> BiSSAP 1.3

<210> 1
<211> 25
<212> DNA
<213> Artificial Sequence

<220>
<223> Primer VH1-FR1

<400> 1
ggcctcagtg aaggtctcct gcaag 25

<210> 2
<211> 25
<212> DNA
<213> Artificial Sequence

<220>
<223> Primer VH2-FR1

<400> 2
gtctggtcct acgctggtga aaccc 25

<210> 3
<211> 25
<212> DNA
<213> Artificial Sequence

<220>
<223> Primer VH3-FR1

<400> 3
ctgggggtc cctgagactc tcctg 25

<210> 4
<211> 25
<212> DNA
<213> Artificial Sequence

<220>
<223> Primer VH4-FR1

<400> 4
cttcggagac cctgtccctc acctg 25

<210> 5
<211> 25
<212> DNA
<213> Artificial Sequence

<220>
<223> Primer VH5-FR1

<400> 5
cggggagtct ctgaagatct cctgt 25

<210> 6
<211> 25
<212> DNA
<213> Artificial Sequence

<220>
<223> Primer VH6-FR1

<400> 6
tcgcagaccc tctcactcac ctgtg 25

<210> 7
<211> 25
<212> DNA
<213> Artificial Sequence

<220>
<223> Primer VH1-FR2

<400> 7
ctgggtgcga caggcccctg gacaa 25

<210> 8
<211> 25
<212> DNA
<213> Artificial Sequence

<220>
<223> Primer VH2-FR2

<400> 8
tggatccgtc agcccccagg gaagg 25

<210> 9
<211> 21
<212> DNA
<213> Artificial Sequence

<220>
<223> Primer VH3-FR2

<400> 9
ggtccgccag gctccaggga a 21

<210> 10
<211> 25
<212> DNA

<213> Artificial Sequence

<220>
<223> Primer VH4-FR2

<400> 10
tggatccgcc agcccccagg gaagg 25

<210> 11
<211> 25
<212> DNA
<213> Artificial Sequence

<220>
<223> Primer VH5-FR2

<400> 11
gggtgcgcca gatgcccggg aaagg 25

<210> 12
<211> 25
<212> DNA
<213> Artificial Sequence

<220>
<223> Primer VH6-FR2

<400> 12
tggatcaggc agtccccatc gagag 25

<210> 13
<211> 25
<212> DNA
<213> Artificial Sequence

<220>
<223> Primer VH7-FR2

<400> 13
ttgggtgcga caggcccctg gacaa 25

<210> 14
<211> 25
<212> DNA
<213> Artificial Sequence

<220>
<223> Primer VH1-FR3

<400> 14
tggagctgag cagcctgaga tctga 25

<210> 15
<211> 25
<212> DNA
<213> Artificial Sequence

<220>

<223> Primer VH2-FR3

<400> 15
caatgaccaa catggaccct gtgga 25

<210> 16
<211> 25
<212> DNA
<213> Artificial Sequence

<220>
<223> Primer VH3-FR3

<400> 16
tctgcaaatg aacagcctga gagcc 25

<210> 17
<211> 25
<212> DNA
<213> Artificial Sequence

<220>
<223> Primer VH4-FR3

<400> 17
gagctctgtg accgccgcgg acacg 25

<210> 18
<211> 25
<212> DNA
<213> Artificial Sequence

<220>
<223> Primer VH5-FR3

<400> 18
cagcaccgcc tacctgcagt ggagc 25

<210> 19
<211> 25
<212> DNA
<213> Artificial Sequence

<220>
<223> Primer VH6-FR3

<400> 19
gttctccctg cagctgaact ctgtg 25

<210> 20
<211> 24
<212> DNA
<213> Artificial Sequence

<220>
<223> Primer VH7-FR3

<400> 20

cagcacggca tatctgcaga tcag 24

<210> 21
<211> 18
<212> DNA
<213> Artificial Sequence

<220>
<223> Primer DH1

<400> 21
ggcggaatgt gtgcaggc 18

<210> 22
<211> 21
<212> DNA
<213> Artificial Sequence

<220>
<223> Primer DH2

<400> 22
gcactgggct cagagtcctc t 21

<210> 23
<211> 20
<212> DNA
<213> Artificial Sequence

<220>
<223> Primer DH3

<400> 23
gtggccctgg gaatataaaa 20

<210> 24
<211> 19
<212> DNA
<213> Artificial Sequence

<220>
<223> Primer DH4

<400> 24
agatccccag gacgcagca 19

<210> 25
<211> 20
<212> DNA
<213> Artificial Sequence

<220>
<223> Primer DH5

<400> 25
cagggggaca ctgtgcatgt 20

<210> 26

<211> 19
<212> DNA
<213> Artificial Sequence

<220>
<223> Primer DH6

<400> 26
tgaccccagc aagggaagg 19

<210> 27
<211> 18
<212> DNA
<213> Artificial Sequence

<220>
<223> Primer DH7

<400> 27
cacaggcccc ctaccagc 18

<210> 28
<211> 22
<212> DNA
<213> Artificial Sequence

<220>
<223> Primer JH57

<400> 28
cttacctgag gagacggtga cc 22

<210> 29
<211> 25
<212> DNA
<213> Artificial Sequence

<220>
<223> Primer VKlf/6

<400> 29
tcaaggttca gcggcagtgg atctg 25

<210> 30
<211> 26
<212> DNA
<213> Artificial Sequence

<220>
<223> Primer VK2f

<400> 30
ggcctccatc tcctgcaggt ctagtc 26

<210> 31
<211> 27
<212> DNA
<213> Artificial Sequence

<220>
<223> Primer VK3f

<400> 31
cccaggctcc tcatctatga tgcatcc 27

<210> 32
<211> 27
<212> DNA
<213> Artificial Sequence

<220>
<223> Primer VK4

<400> 32
caactgcaag tccagccaga gtgtttt 27

<210> 33
<211> 26
<212> DNA
<213> Artificial Sequence

<220>
<223> Primer VK5

<400> 33
cctgcaaagc cagccaagac attgat 26

<210> 34
<211> 27
<212> DNA
<213> Artificial Sequence

<220>
<223> Primer VK6

<400> 34
gaccgatttc accctcacaa ttaatcc 27

<210> 35
<211> 27
<212> DNA
<213> Artificial Sequence

<220>
<223> Primer JK1-4

<400> 35
cttacgtttg atctccacct tggtccc 27

<210> 36
<211> 27
<212> DNA
<213> Artificial Sequence

<220>
<223> Primer JK5

<400> 36
cttacgttta atctccagtc gtgtccc 27

<210> 37
<211> 27
<212> DNA
<213> Artificial Sequence

<220>
<223> Primer Kde

<400> 37
cctcagaggt cagagcaggt tgtccta 27

<210> 38
<211> 22
<212> DNA
<213> Artificial Sequence

<220>
<223> Primer INTR

<400> 38
cgtggcaccg cgagctgtag ac 22